(19) Europäisches Patentamt / European Patent Office / Office européen des brevets

(11) **EP 4 361 624 B1**

(12) **EUROPEAN PATENT SPECIFICATION**

(45) Date of publication and mention of the grant of the patent:
**06.05.2026 Bulletin 2026/19**

(21) Application number: **22828206.7**

(22) Date of filing: **06.06.2022**

(51) International Patent Classification (IPC):
**G16C 20/20** (2019.01)    **G01N 27/62** (2021.01)
**H01J 49/00** (2006.01)

(52) Cooperative Patent Classification (CPC):
**H01J 49/0036; G16C 20/20**

(86) International application number:
**PCT/JP2022/022813**

(87) International publication number:
**WO 2022/270289 (29.12.2022 Gazette 2022/52)**

(54) **METHOD FOR ESTIMATING CONTENT RATIO OF COMPONENTS CONTAINED IN SAMPLE, COMPOSITION ESTIMATING DEVICE, AND PROGRAM**

VERFAHREN ZUR SCHÄTZUNG DES INHALTSVERHÄLTNISSES VON IN EINER PROBE ENTHALTENEN KOMPONENTEN, ZUSAMMENSETZUNGSSCHÄTZVORRICHTUNG UND PROGRAMM

PROCÉDÉ D'ESTIMATION DE LA TENEUR EN COMPOSANTS CONTENUS DANS UN ÉCHANTILLON, DISPOSITIF D'ESTIMATION DE COMPOSITION ET PROGRAMME

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **24.06.2021 JP 2021104510**

(43) Date of publication of application:
**01.05.2024 Bulletin 2024/18**

(73) Proprietor: **National Institute for Materials Science**
**Tsukuba-shi, Ibaraki 305-0047 (JP)**

(72) Inventors:
• **NAITO, Masanobu**
  **Tsukuba-shi, Ibaraki 305-0047 (JP)**
• **HIBI, Yusuke**
  **Tsukuba-shi, Ibaraki 305-0047 (JP)**

(74) Representative: **J A Kemp LLP**
**80 Turnmill Street**
**London EC1M 5QU (GB)**

(56) References cited:
**WO-A1-2016/120958    WO-A1-2018/158801**
**JP-A- 2012 516 445    JP-A- 2013 528 287**

**JP-A- 2021 081 365    US-A1- 2007 288 174**
**US-A1- 2009 121 125**

• **KOPRIVA I ET AL: "Blind Separation of Analytes in Nuclear Magnetic Resonance Spectroscopy and Mass Spectrometry: Sparseness-Based Robust Multicomponent Analysis", ANALYTICAL CHEMISTRY, AMERICAN CHEMICAL SOCIETY, vol. 82, no. 5, 1 March 2010 (2010-03-01), pages 1911 - 1920, XP002571604, DOI: 10.1021/ AC902640Y**
• **KOPRIVA I ET AL: "Multi-component analysis: blind extraction of pure components mass spectra using sparse component analysis", JOURNAL OF MASS SPECTROMETRY, WILEY CHICHESTER, GB, vol. 44, 1 September 2009 (2009-09-01), pages 1378 - 1388, XP002571605, ISSN: 1076-5174, [retrieved on 20090807], DOI: 10.1002/JMS.1627**
• **RAPIN J�R�MY ET AL: "Application of non-negative matrix factorization to LC/MS data", SIGNAL PROCESSING, vol. 123, 7 January 2016 (2016-01-07), pages 75 - 83, XP029429847, ISSN: 0165-1684, DOI: 10.1016/J.SIGPRO.2015.12.014**

## Description

Technical Field

The present invention relates to a method of inferring a content ratio of a component in a sample, a composition inference device, and a program.

Background Art

As a method for inferring a component composition in a sample as a multicomponent mixing system, there is a conventionally-known method of separating components in the sample by some process and identifying and quantifying each fractionated component. One of such identifying methods uses a mass spectrum acquired by a mass spectrometer.

However, information acquired from mass spectra is a huge volume of multidimensional data having a large number of correlations, imposing difficulty in extracting a distinctive signal leading to component identification intuitively or empirically. As described in Non-Patent Literature 1, a method of searching for a feature by reducing the dimension of such multidimensional data through main component analysis has been used recently. However, even such known data analysis technique is used, it is still inherently difficult to realize quantification using a mass spectrum having a peak intensity depending on ionization efficiency. For quantitative analysis, it is first required to determine ionization efficiency that is an unknown variable for each compound. To achieve this, isotopic samples having known sample volumes and having the same ionization efficiency should be used as benchmark materials.

US 2007/0288174 A1 discloses as follows. Namely, "A system is provided for analyzing metabolomics data received from an analytical device across a group of samples. The system automatically receives a data matrix corresponding to each of the samples, wherein the data matrix includes rows corresponding to each of the samples and columns corresponding to a group of ions present in the respective samples. A processor is provided for determining a characteristic value corresponding to at least one of a group of components present in the samples, wherein the components are made up of at least a portion of the group of ions, using at least one of a correlation function and a factorization function. A user interface is in communication with the processor for displaying a visual indication of the characteristic value such that a user may receive a visual indication of the types of components present in the samples."

US 2009/0121125 A1 discloses as follows. Namely, "A method of obtaining pure component mass spectra or pure peak elution profiles from mass spectra of a mixture of components involves estimating number of components in the mixture, filtering noise, and extracting individual component mass spectra or pure peak elution profiles using blind entropy minimization with direct optimization (e.g. downhill simplex minimization). The method may be applied to deconvolution of pure GC/MS spectra of overlapping or partially overlapping isotopologues or other compounds, separation of overlapping or partially overlapping compounds in proteomics or metabolomics mass spectrometry applications, peptide sequencing using high voltage fragmentation followed by deconvolution of the obtained mixture mass spectra, deconvolution of MALDI mass spectra in the separation of multiple components present in a single solution, and specific compound monitoring in security and/or environmentally sensitive areas."

Non-Patent Literature 2 discloses as follows. Namely, "Metabolic profiling of biological samples involves nuclear magnetic resonance (NMR) spectroscopy and mass spectrometry coupled with powerful statistical tools for complex data analysis. Here, we report a robust, sparseness-based method for the blind separation of analytes from mixtures recorded in spectroscopic and spectrometric measurements. The advantage of the proposed method in comparison to alternative blind decomposition schemes is that it is capable of estimating the number of analytes, their concentrations, and the analytes themselves from available mixtures only. The number of analytes can be less than, equal to, or greater than the number of mixtures. The method is exemplified on blind extraction of four analytes from three mixtures in 2D NMR spectroscopy and five analytes from two mixtures in mass spectrometry. The proposed methodology is of widespread significance for natural products research and the field of metabolic studies, whereupon mixtures represent samples isolated from biological fluids or tissue extracts."

Citation List

Non-Patent Literature

Non-Patent Literature 1: Analytical chemistry, 2020, vol. 92, issue 2, pp. 1925-1933

Non-Patent Literature 2: Ivica Kopriva and Ivanka Jeric, Analytical Chemistry, Vol. 82, pp. 1911-1920 (2010)

Summary of Invention

Technical Problem

According to the method described in Non-Patent Literature 1, much of information contained in the original multidimensional data is lost during the course of the dimension reduction. This disables quantitative analysis on a component even through qualitative analysis thereon is possible.

Furthermore, as long as there is the characteristic that ionization efficiency differs between components in response to a component type or a test environment, etc., a ratio between the sizes (areas) of resultant mass spectra does not reflect a content ratio of each component in a mixture. This forms one of factors to inhibit quantitative analysis using mass spectra.

Ambient desorption ionization (ADI) has widely been used recently as one of the mass spectrometry methods by which a specimen in the ambient condition (in the open air under atmospheric pressure) is subjected to mass spectrometry without preprocessing. While this method has an advantage in terms of allowing unprocessed specimens or open-system specimens to be analyzed as they are in solid state, it has been deemed to make quantitative analysis more difficult due to influence by inconsistence of ionization rate between components, and the measurement environment, etc.

In consideration of the foregoing circumstances, an object of the present invention is to provide a component content ratio inferring method capable of inferring a composition of components in an unknown mixture even from a mass spectrum acquired under the ambient condition.

It is also an object of the present invention to provide a composition inference device and a program.

Means of Solving Problem

The present inventors conducted study earnestly to attain the foregoing objects, and found that these objects can be attained by configurations described below.

[1] A method of inferring a content ratio of a component in an inference target sample containing at least one type of the component selected from K types of components while K is an integer equal to or greater than 1. The method includes: preparing learning samples of a number equal to or greater than K containing at least one type of component selected from the K types of components and having compositions different from each other, and a background sample not containing the component; sequentially ionizing gas components generated by thermal desorption and/or pyrolysis while heating each sample in a sample set including the inference target sample, the learning samples, and the background sample, and observing mass spectra continuously; storing, into respective rows, mass spectra acquired for a plurality of heating temperatures to acquire two-dimensional mass spectra of the respective samples, and merging at least two or more of the two-dimensional spectra and converting the spectra into a data matrix; performing NMF process by which the data matrix is subjected to non-negative matrix factorization to be factorized into the product of a normalized base spectrum matrix and a corresponding intensity distribution matrix; extracting a noise component in the intensity distribution matrix through analysis on canonical correlation between the base spectrum matrix and the data matrix, and correcting the intensity distribution matrix so as to reduce influence by the noise component, thereby acquiring a corrected intensity distribution matrix; partitioning the corrected intensity distribution matrix into a submatrix corresponding to each of the samples, and expressing each of the samples in vector space using the submatrix as a feature vector; defining a K-1 dimensional simplex including all of the feature vectors and determining K end members in the K-1 dimensional simplex; and calculating an Euclidean distance between each of the K end members and the feature vector of the inference target sample, and inferring a content ratio of the component in the inference target sample on the basis of a ratio of the Euclidean distance. If the K is equal to or greater than 3, at least one of the feature vectors of the learning samples is present in each region external to a hypersphere inscribed in the K-1 dimensional simplex or the learning samples contain at least one of the end members.

[2] The method according to [1], wherein the K is an integer equal to or greater than 2, and the inference target sample is a mixture of the components.

[3] The method according to [1] or [2], wherein the learning sample contains the end member.

[4] The method according to [3], wherein the end member is determined on the basis of a determination label given to the learning sample.

[5] The method according to [3], wherein the end member is determined through vertex component analysis on the feature vector of the learning sample.

[6] The method according to [1], wherein the end member is determined on the basis of an algorithm by which a vertex is defined in such a manner that the K-1 dimensional simplex has a minimum volume.

[7] The method according to [6], wherein the end member is determined by second NMF process by which the corrected intensity distribution matrix is subjected to non-negative matrix factorization to be factorized into the product of a matrix representing the weight fractions of the K types of components in the sample and a matrix representing an individual fragment abundance of each of the K types of components.

[8] The method according to [6] or [7], wherein the learning sample does not contain the end member.

[9] The method according to any one of [1] to [8], wherein acquiring the corrected intensity distribution matrix further includes making intensity correction on the intensity distribution matrix.

[10] The method according to [9], wherein the sample set further includes a calibration sample, the calibration sample contains all the K types of components and has a known composition, and the intensity correction includes normalizing the intensity distribution matrix.

[11] The method according to [9], wherein the intensity correction includes allocating at least part of the intensity distribution matrix using the product of the mass of the corresponding sample in the sample set and an environmental variable, and the environmental variable is a variable representing influence on ionization efficiency of the component during the observation.

[12] The method according to [11], wherein the environmental variable is a total value of peaks in a mass spectrum of a compound having a molecular weight of 50-1500 contained in a predetermined quantity in an atmosphere during the observation or an organic low-molecular compound having a molecular weight of 50-500 contained in a predetermined quantity in each sample in the sample set.

[13] A composition inference device that infers a content ratio of a component in an inference target sample containing at least one type of the component selected from K types of components while K is an integer equal to or greater than 1. The composition inference device includes: a mass spectrometer that sequentially ionizes gas components generated by thermal desorption and/or pyrolysis while heating each of samples in a sample set including learning samples of a number equal to or greater than K containing at least one type of component selected from the K types of components and having compositions different from each other, a background sample not containing the component, and the inference target sample, and observes mass spectra continuously; and an information processing device that processes the observed mass spectra. The information processing device includes: a data matrix generating part that stores, into respective rows, mass spectra acquired for a plurality of heating temperatures to acquire two-dimensional mass spectra of the respective samples, and merges at least two or more of the two-dimensional spectra and converts the spectra into a data matrix; an NMF processing part that performs NMF process by which the data matrix is subjected to nonnegative matrix factorization to be factorized into the product of a normalized base spectrum matrix and a corresponding intensity distribution matrix; a correction processing part that extracts a noise component in the intensity distribution matrix through analysis on canonical correlation between the base spectrum matrix and the data matrix, and corrects the intensity distribution matrix so as to reduce influence by the noise component, thereby generating a corrected intensity distribution matrix; a vector processing part that partitions the corrected intensity distribution matrix into a submatrix corresponding to each of the samples in the sample set, and expresses each of the samples in vector space using the submatrix as a feature vector; an end member determining part that defines a K-1 dimensional simplex including all of the feature vectors and determines K end members in the K-1 dimensional simplex; and a content ratio calculating part that calculates an Euclidean distance between each of the K end members and the feature vector of the inference target sample, and infers a content ratio of the component in the inference target sample on the basis of a ratio of the Euclidean distance. If the K is equal to or greater than 3, at least one of the feature vectors of the learning samples is present in each region external to a hypersphere inscribed in the K-1 dimensional simplex or the learning samples contain at least one of the end members.

[14] The composition inference device according to [13], wherein the end member determining part determines the end member on the basis of a determination label given to the learning sample.

[15] The composition inference device according to [13], wherein the end member determining part determines the end member on the basis of an algorithm by which a vertex is defined in such a manner that the K-1 dimensional simplex has a minimum volume.

[16] The composition inference device according to [15], wherein the end member determining part determines the end member by performing second NMF process by which the corrected intensity distribution matrix is subjected to non-negative matrix factorization to be factorized into the product of a matrix representing the weight fractions of the K types of components in the sample and a matrix representing an individual fragment abundance of each of the K types of components.

[17] The composition inference device according to [15], wherein the correction processing part further makes intensity correction on the intensity distribution matrix.

[18] The composition inference device according to [17], wherein the sample set further includes a calibration sample, the calibration sample contains all the K types of components and has a known composition, and the intensity correction includes normalizing the intensity distribution matrix.

[19] The composition inference device according to [17], wherein the intensity correction includes allocating at least part of the intensity distribution matrix using the product of the mass of the corresponding sample in the sample set and an environmental variable, and the environmental variable is a variable representing influence on ionization efficiency of the component during the observation.

[20] The composition inference device according to [19], wherein the mass spectrometer further includes a pressure-reducing pump, and the environmental variable is a total value of peaks in a mass spectrum of a material generated by operation of the pressure-reducing pump.

[21] A program used in a composition inference device that infers a content ratio of a component in an inference target sample containing at least one type of the component selected from K types of components while K is an integer equal to or greater than 1. The composition inference device includes: a mass spectrometer that sequentially ionizes gas components generated by thermal desorption and/or pyrolysis while heating each of samples in a sample set including learning samples of a number equal to or greater than K containing at least one type of component selected from the K types of components and having compositions different from each other, a background sample not containing the component, and the inference target sample, and observes mass spectra continuously; and an information processing device that processes the observed mass spectra. The program includes: a data matrix generating function of storing, into respective rows, mass spectra acquired for a plurality of heating temperatures by the mass spectrometer to acquire two-dimensional mass spectra of the respective samples, and merging at least two or more of the two-dimensional spectra and converting the spectra into a data matrix; an NMF processing function of performing NMF process by which the data matrix is subjected to non-negative matrix factorization to be factorized into the product of a normalized base spectrum matrix and a corresponding intensity distribution matrix; a correction processing function of extracting a noise component in the intensity distribution matrix through analysis on canonical correlation between the base spectrum matrix and the data matrix, and correcting the intensity distribution matrix so as to reduce influence by the noise component, thereby generating a corrected intensity distribution matrix; a vector processing function of partitioning the corrected intensity distribution matrix into a submatrix corresponding to each of the samples, and expressing each of the samples in vector space using the submatrix as a feature vector; an end member determining function of defining a K-1 dimensional simplex including all of the feature vectors and determining K end members in the K-1 dimensional simplex; and a content ratio calculating function of calculating an Euclidean distance between each of the K end members and the feature vector of the inference target sample, and inferring a content ratio of the component in the inference target sample on the basis of a ratio of the Euclidean distance. If the K is equal to or greater than 3, at least one of the feature vectors of the learning samples is present in each region external to a hypersphere inscribed in the K-1 dimensional simplex or the learning samples contain at least one of the end members.

Advantageous Effects of Invention

One of characteristics of the method of the present invention (hereinafter also called a "present method") is that, gas components generated by thermal desorption and/or pyrolysis are ionized sequentially while each sample in the sample set is heated and mass spectra are observed continuously, the mass spectrum acquired for each heating temperature is stored into each row to acquire two-dimensional mass spectra of the respective samples, these spectra are merged and converted into a data matrix, the data matrix is subjected to non-negative matrix factorization (NMF) to be factorized into the product of the normalized base spectrum matrix and the corresponding intensity distribution matrix, a noise component in the intensity distribution matrix is extracted through analysis on canonical correlation and the intensity distribution matrix is corrected, a corrected intensity distribution matrix containing a feature vector of each sample is acquired, and quantitative calculation is performed thereafter.

By performing the process of correcting the intensity distribution matrix through the canonical correlation analysis, it becomes possible to make quantitative analysis more easily with high precision (the details thereof will be described later).

If the K is an integer equal to or greater than 2 and a sample as an inference target (inference target sample) is a mixture of the K types of components, the present method makes it possible to infer a mixture ratio of the K types of components (composition) in the inference target sample.

If the learning sample contains the end member, it is possible to infer the composition of the sample more accurately and more easily.

If the learning sample is given the determination label for determining the end member, the end member can be determined on the basis of information about the label, making it possible to infer the composition of the sample more easily.

If the determination label is not given and the learning sample contains the end member, the end member can be determined through vertex component analysis on the feature vector of the learning sample, allowing inference of the composition of the sample.

Even if the sample does not contain the end member, the composition of the sample can still be inferred by determining the end member using an algorithm by which a vertex is defined in such a manner that the K-1 dimensional simplex has a minimum volume. This has excellence in that it does not require the end member as an essential in the learning sample.

If the corrected intensity distribution matrix is a matrix after the intensity correction on the intensity distribution matrix, the accuracy of inference is increased further.

If the sample set further includes the calibration sample and the intensity correction includes normalizing the intensity distribution matrix, the composition of the sample can be inferred more precisely by a simpler procedure.

By allocating part of the intensity distribution matrix corresponding to each sample using the product of the mass of the sample and the environmental variable, influence by the ionization efficiency of each component during the observation is corrected. Thus, even in the absence of the calibration sample in the sample set, it is still possible to infer the composition of the sample with high precision.

If the environmental variable used in the foregoing allocation is a total value of peaks in a mass spectrum of a compound having a molecular weight of 50-1500 contained in a predetermined quantity in an atmosphere during the observation or an organic low-molecular compound having a molecular weight of 50-500 contained in a predetermined quantity in each sample in the sample set, it is possible to acquire more accurate composition inference result through simpler operation even in the absence of the calibration sample in the sample set.

Brief Description of Drawings

Fig. 1 is a flowchart of a composition inference method according to an embodiment of the present invention;

Fig. 2 is a conceptual view showing a K-1 dimensional simplex (two-dimensional simplex, a triangle) while K is 3;

Fig. 3 is a hardware configuration view of a composition inference device according to the embodiment of the present invention;

Fig. 4 is a functional block diagram of the composition inference device according to the embodiment of the present invention;

Fig. 5 is a view showing an original spectrum (A) of "MSE_calib" and a spectrum (B) reconstructed after process;

Fig. 6 shows $C_{\delta(MSE\_Calib)}$ as part of a matrix C after NMF factorization and $S^T$ resulting from removing a background spectrum and an impurity spectrum using CCA-Filter;

Fig. 7 shows composition analysis result about a ternary system composed of M (poly(methyl methacrylate)), S (polystyrene), and E (poly(ethyl methacrylate));

Fig. 8 shows composition inference result obtained by eliminating orthogonal constraint from an NMF condition;

Fig. 9 shows composition inference result obtained by eliminating only merging S from a condition for NMF optimization;

Fig. 10 shows composition inference result corresponding to result obtained by eliminating CCA-filter (Comparative Example);

Fig. 11 is a picture showing an original spectrum (raw of MSE_calib in the upper section) of "MSE_calib" and a spectrum reconstructed after eliminating CCA-filter and then performing process;

Fig. 12 shows composition inference result obtained without making correction using an environmental variable;

Fig. 13 is a conceptual view showing a K-1 dimensional simplex (two-dimensional simplex, a triangle) while K is 3;

Fig. 14 is an explanatory view of a mechanism of a method of determining an end member according to a second embodiment of the present invention;

Fig. 15 is a view showing inference result according to an Example 2; and

Fig. 16 is a view showing inference result according to an Example 3.

Description of Embodiments

The present invention will be described below in detail.

While constituting elements will be described below on the basis of representative embodiments of the present invention, the present invention are not limited to these embodiments.

In the present description, a numerical value range expressed using "-" means a range including numerical values written before and after "-" as a lower limit value and an upper limit value.

[Definition of Terms]

Terms used in the present description will be explained. A term to be used and not to be explained below will be given a meaning generally understandable for persons skilled in the art.

In the present description, a "component" means a material forming each sample, and an inference target sample, a learning sample, and a calibration sample each mean a mixture composed of a combination of components.

The "inference target sample" means a sample to be subjected to composition inference, and the number of components and the content of each component therein may be unknown.

The "learning sample" means samples required in determining in K end members in a K-1 dimensional simplex described later. These samples contain at least one type of component selected from K types of components and have different composition. The number of the contained components is not particularly limited but may be 1-K types. The learning samples may include a sample having the same composition as the inference target sample. Specifically, while the inference target sample and the learning sample may be the same, the learning samples differ from each other.

The "end member" means a vector corresponding to a vertex of the K-1 dimensional simplex. This end member corresponds to a feature vector of a sample composed of only one of the K types of components (learning sample or virtual sample).

A "background sample" means a sample different from the inference target sample and the learning sample, it means a sample containing none of the foregoing components.

The "calibration sample" means a sample different from the inference target sample, the learning sample, and the background sample, it means a sample containing all the K types of components and having a definite composition.

[First Embodiment of Present Method]

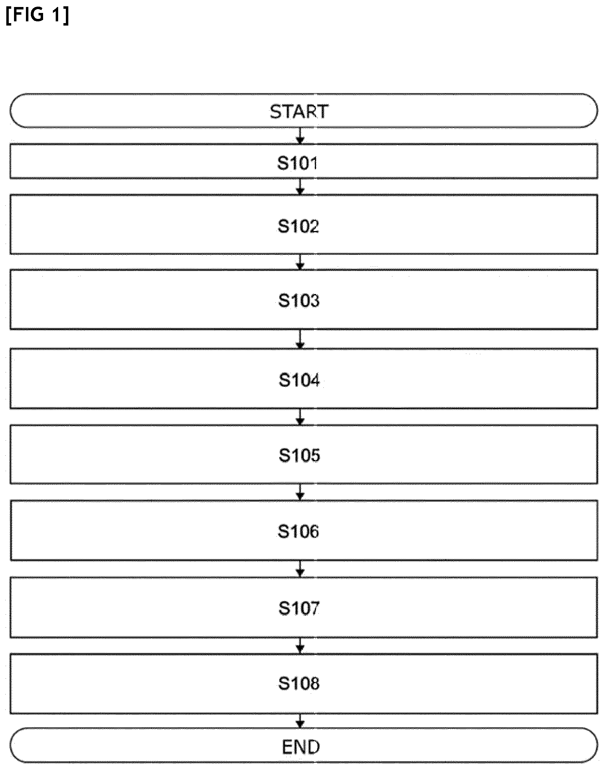

Fig. 1 is a flowchart of a composition inference method (hereinafter also called a "present method") according to an embodiment of the present invention.

First, an inference target sample, learning samples of a number of equal to or greater than K having compositions different from each other, and a background sample are prepared (these samples will collectively be called a "sample set") (step S101). The sample set may further include a calibration sample.

The inference target sample contains at least one type of component among the K types of components. If the inference target sample contains one type of component, the purity of this component can be inferred by the present method. In this case, the inference target sample contains this "component" and impurities other than this component.

If K is equal to or greater than 2, the inference target sample is a mixture of components. As long as K is equal to or greater than 1 in the present method, an upper limit thereof is not particularly limited. Typically, K is preferably equal to or less than 1000.

The number of the learning samples relates to the number K of components and is required to be equal to or greater than K. The number of the learning samples is not particularly limited as long as it is equal to or greater than K. Meanwhile, with a larger number of the learning samples, a vertex of the K-1 dimensional simplex described later is likely to be determined with higher precision. This eventually facilitates acquisition of more accurate composition inference result.

Specifically, the ratio of the number of the learning samples to the number K of components (the number of learning samples/the number of components) is equal to or greater than 1, preferably, equal to or greater than 2, more preferably, equal to or greater than 3, still more preferably, equal to or greater than 5, particularly preferably, equal to or greater than 10. Meanwhile, while an upper limit of the ratio is not particularly limited, it is generally preferably equal to or less than 1000.

The learning samples having compositions different from each other. The composition is determined by the type of a contained component, the number of the components, and the content of each component. Samples having the same composition means that these samples are the same in terms of the type of a contained component, the number of components, and the content of each component. If any one of these elements differs between the samples, these samples have "compositions different from each other."

According to the present method, as long as the learning samples have compositions different from each other, information other than the difference, specifically, the type of a contained component, the number of the components, and the content of each component are not necessary for composition inference of the inference target sample. Namely, the composition of the learning sample may be unknown.

A difference between the inference target sample and the learning sample is that the compositions of the learning samples should differ from each other. The learning samples may include a learning sample having the same composition as the inference target sample.

The learning sample may contain an end member. Specifically, the learning sample may contain a member composed of one type of component (single-ingredient, pure) selected from the K types of components. If the learning sample contains the end member, high-precision composition inference can be made more easily.

In this case, the learning sample as the end member may contain a determination label. The learning sample preferably contains the determination label as it allows the end member to be determined more easily.

Next, each inference target sample is heated to sequentially ionize gases generated by thermal desorption and/or pyrolysis, thereby acquiring a mass spectrum (step S102).

While a method of acquiring the mass spectrum is not particularly limited, a method of performing mass spectrometry without preparatory process on a specimen in an ambient condition is preferred. A mass spectrometer called DART-MS using an ion source called a DART (direct analysis in real time) ion source and a mass spectrometer instrument in combination is known as a device used in a method for the ionization and mass spectrometry described above.

The mass spectrometer instrument is not particularly limited but is preferably an instrument allowing precise mass spectrometry and may be any of types including a quadrupolar type and a time-of-flight (TOF) type.

While a condition for acquisition of the mass spectrum is not particularly limited, according to a procedure given as a non-restrictive example, all the samples in the sample set are sequentially heated at a temperature increasing rate of 50°C/min, and helium ions are injected at an interval of 50 shot/min to pyrolysis gas generated in a temperature range of 50-550°C to ionize the gas, thereby acquiring a two-dimensional MS spectrum having m/z along a horizontal axis and a temperature along a vertical axis.

Next, the mass spectrum acquired at each heating temperature is stored in each row to acquire a two-dimensional mass spectrum of each sample, and at least two or more of these two-dimensional mass spectra are merged and converted to a data matrix (step S103).

A method of acquiring the data matrix will be described in detail.

In step S102, mass spectra are acquired continuously on the basis of each predetermined temperature increasing interval. While these mass spectra can be used as they are in generating the data matrix, they may be used after being averaged on the basis of each predetermined temperature increasing range. Averaging the mass spectra on the basis of each predetermined temperature increasing range and merging the mass spectra into one allows compression of a data volume. This temperature increasing range is approximately 10-30°C, for example.

In each spectrum, a peak intensity may be normalized. For example, this normalization can be realized by a method of normalizing peak intensities in such a manner that the sum of squares of the peak intensities becomes 1.

As a result, by making one measurement on one sample, it becomes possible to acquire mass spectra of a predetermined number at each heating temperature (or heating temperatures merged in each predetermined range) (this number of the mass spectra differs depending on a way of the merging and may be 20, for example).

By storing each of these mass spectra in each row and the heating temperature in each column, a two-dimensional mass spectrum is acquired for each sample.

After the two-dimensional mass spectra are acquired for the respective samples in this way, at least two or more of these spectra are merged and converted to a data matrix X.

As long as the number of the two-dimensional mass spectra used in generating the data matrix X is equal to or greater than 2, it is not particularly limited. Meanwhile, it is preferable that two-dimensional mass spectra of all the samples in the sample set be used.

If one sample is to be subjected to measurements twice or more, some or all of two-dimensional mass spectra acquired by the two or more measurements may be used in generating the data matrix X.

Next, the acquired data matrix is subjected to non-negative matrix factorization (NMF) to be factorized into the product of a normalized base spectrum matrix and a corresponding intensity distribution matrix (step S104).

The non-negative matrix factorization will be described.

Under the assumption that linear additive property is established between an intensity of a spectrum and a component composition of a sample, a data matrix of the spectrum can generally be subjected to matrix decomposition as

[Formula 1]

$$X = CS^T$$

Here, the data matrix X is a data matrix of $N \times M$ obtained by piling N spectra, each having M numbers of channels, S is a base spectrum matrix, and C is a corresponding intensity distribution matrix. Assuming that the number of all components contained in a data set is $K_1$, C is a matrix $N \times K_1$ and S is a matrix $M \times K_1$. As noise is present in actual measurement,

[Formula 2]

$$X = CS^T + N$$

by taking an error matrix N into consideration in this way, the C and S matrices are defined in such a manner as to minimize N. A sign T on the upper right side of the matrix means a transposed matrix. Here, on the assumption of the existence of Gaussian noise,

[Formula 3]

$$\min_{C, S} \left\| X - CS^T \right\|_F^2$$

is formulated as an optimization problem about C and S. Furthermore,

[Formula 4]

$$\left\| matix \right\|_F$$

represents a matrix Frobenius norm,

[Formula 5]

$$\|vec\|_1$$

represents an L1 norm of a vector, and

[Formula 6]

$$\|vec\|$$

represents an L2 norm of the vector.

As C and S in the foregoing formulas do not have unique solutions, a restraint condition assumed to be satisfied by a spectrum in response to physical requirement is imposed. On the basis of obvious requirement that a composition ratio or a spectrum intensity is to take a non-negative value, the NMF is formulated as

[Formula 7]

$$\min_{C \geq 0, \quad S \geq 0} \|X - CS^T\|_F^2$$

However, as a unique solution is not defined only under a non-negative condition, it is required to apply an additional restraint condition responsive to a measuring device. A restraint condition for NMF in the present step will be described below.

(NMF Update formula)

Preferably, in the present step, the NMF process is performed to solve the foregoing formula (formula 7) as a convex optimization problem with respect to each column of C and S by making updates alternately between C and S in a column vectorwise manner using hierarchical alternating least square (HALS) into which constraint mentioned below can be incorporated easily.

According to the present method, mass spectrometry is performed on gas generated by thermal desorption and/or pyrolysis while each sample is heated. Hence, the number $K_1$ of components after the pyrolysis is hard to acquire as prior information and should be inferred from data accordingly. As a method of the inference, automatic relevance determination (ARD) is preferred. This method is to assume sparseness in the intensity distribution matrix C. This assumption can be deemed to be natural considering that pyrolysis of one component occurs only at a particular temperature of a sample containing this component. By doing so, balance is obtained between power of minimizing the number of components and power of minimizing the foregoing formula (formula 7), making it possible to determine an optimum number of components.

(Orthogonal Constraint)

A mass spectrometer instrument generally has a resolution that is approximately 0.1-0.001 m/z. This makes the occurrence of a situation rare where different gas components are equal to each other to a place after the decimal point in terms of m/z and share the same channel. For this reason, it is reasonable to assume orthogonality to a certain degree between column vectors of the base spectrum matrix S.

This not only achieves the effect of resulting in a favorable local solution but is also expected to achieve the effect of preventing concentration of fitting only on a high intensity peak. On the other hand, complete orthogonality and non-negativity result in excessively severe constraint. For this reason, it is preferable that orthogonal constraint be imposed softly by adding a penalty term having a Lagrange's undetermined multiplier (soft orthogonal constraint).

(Component Merging)

If two or more base spectra nearly equal to each other are acquired during the course of updating C and S, a correct number n of components can be acquired by merging these spectra into one spectrum. By applying EALS spectra images to the NMF and writing a k-th column vector in S as $S_{:k}$, in the presence of k < m that satisfies $S^T_{:k}S_{:m}$ > threshold, merging can be conducted as (Merging C):

[Formula 8]

$$C_{:k} \leftarrow C_{:k} + C_{:m}$$

$$C_{:m} \leftarrow 0$$

Here, a threshold is a hyper parameter given in a range of 0.9-0.99. Preferably, merging is performed on similar S in the same way. Still preferably, if C has a plurality of column vectors parallel to each other, corresponding column vectors in S are superimposed in response to the weight of C. Specifically,

[Formula 9]

$$C_{:k}^{T} C_{:m} > threshold \|C_{:k}\| \|C_{:m}\|$$

in the presence of k ≠ m satisfying this formula, merging (Merging S) is performed as

[Formula 10]

$$S_{:k} \leftarrow S_{:k} + \frac{\|C_{:m}\|_1}{\|C_{:k}\|_1} S_{:m}$$

$$s = \|S_{:k}\|$$

$$S_{:k} \leftarrow \frac{S_k}{\|S_{:k}\|}$$

$$C_{:k} \leftarrow s C_{:k}$$

$$C_{:m} \leftarrow 0$$

This is expected to achieve the effect of merging isotopic peaks or oligomer peaks into one base spectrum.

(Generation of Update formula)

The update formula obtained by the foregoing method will be described.

A totally simultaneous distribution of a model is

[Formula 11]

$$p(X, C, S) = p(X|C, S, \sigma^2)p(C|\lambda)p(S)p(\lambda|a, b)$$

where $\sigma^2$ is a variance parameter.

[Formula 12]

$$\lambda \in \mathbb{R}^K$$

An element $\lambda_k$ in this formula is a parameter following an inverse gamma distribution representing the significance of a component k in data. As noise is assumed to have a Gaussian distribution,

[Formula 13]

$$p(X|C, S) = \prod_{i=1}^{N} \prod_{j=1}^{M} \frac{1}{\sqrt{2\pi\sigma^2}} \exp\left\{-\frac{(X_{ij} - [CS^T]_{ij})^2}{2\sigma^2}\right\}$$

Assuming that K is the number of components significantly large (about 50) to be input as an initial value, an exponential distribution to be followed by C is

[Formula 14]

$$p(C|\lambda) = \prod_{i=1}^{N} \prod_{k=1}^{K} p(C_{ik}|\lambda_k)$$

$$p(C_{ik}|\lambda_k) = \frac{1}{\lambda_k} \exp\left(-\frac{C_{ik}}{\lambda_k}\right)$$

.

An inverse gamma distribution to be followed by $\lambda_k$ is

[Formula 15]

$$p(\lambda|a,b) = \prod_{k=1}^{K} p(\lambda_k|a,b) = \frac{b^a}{\Gamma(a)} \lambda_k^{-(a+1)} \exp\left(-\frac{b}{\lambda_k}\right), k = 1, \ldots, K$$

Here, a is a hyper parameter with which a-1 becomes an extremely small positive number, and b is a parameter defined as follows using an average $\mu_x$ of data matrices:

[Formula 16]

$$b = \frac{\mu_X(a-1)\sqrt{N}}{K}$$

Furthermore, an expected value of an element in C is

[Formula 17]

$$E[C_{ik}] = \frac{\mu_X \sqrt{N}}{K}$$

.

A negative log likelihood of p(X, C, S) is

[Formula 18]

$$L(X, C, S, \lambda) = -\log[p(X|C, S)p(C|\lambda)p(\lambda|a, b)]$$

$$= \frac{1}{2\sigma^2}\|X - CS^T\|_F^2 + \frac{MN}{2}log2\pi\sigma^2 + (N + a + 1)\sum_{k=1}^{K}log\lambda_k$$

$$+ \sum_{k=1}^{K}\frac{1}{\lambda_k}(b + \sum_{i=1}^{N}C_{ik}) + K(log\Gamma(a) - alogb)$$

Here, the variance parameter $\sigma^2$ under the assumption of a Gaussian distribution is given as

[Formula 19]

$$\sigma^2 = \frac{1}{MN}\|X - CS^T\|_F^2$$

Furthermore, as a convex function is determined for λ,

[Formula 20]

$$\lambda_k = \frac{b + \sum_{i=1}^{N} C_{ik}}{N + a + 1}$$

can be obtained.

Next, an update formula for S will be described. Extracting only a term $J(S_{:k})$ related to $S_{:k}$ from a negative log likelihood function is considered first.

[Formula 21]

$$X^{(k)} = X - CS^T + C_{:k}S_{:k}^T$$

Using a contribution of a component k to X written in this way,

[Formula 22]

$$\|X - CS^T\|_F^2 = \|X^{(k)} - C_{:k}S_{:k}^T\|_F^2 = Tr[(X^{(k)} - C_{:k}S_{:k}^T)^T (X^{(k)} - C_{:k}S_{:k}^T)]$$

can be defined. Then, by removing a constant term,

[Formula 23]

$$J(S_{:k}) = \frac{1}{2\sigma^2}[-2S_{:k}^T X^{(k)} C_{:k} + \|C_{:k}\|^2 \|S_{:k}\|^2]$$

This formula was derived on the basis of the following: Shiga, M. et al., Sparse modeling of EELS and EDX spectral imaging data by nonnegative matrix factorization. Ultramicroscopy 170, 43-59 (2016). Here, a penalty term to be added to S is incorporated that is determined by the foregoing orthogonal constraint added to S.

[Formula 24]

$$J(S_{:k}) = \frac{1}{2\sigma^2}[-2S_{:k}^T X^{(k)\,T} C_{:k} + \|C_{:k}\|^2 \|S_{:k}\|^2] + \xi_k s^{(k)\,T} S_{:k}$$

Here, $\xi_k$ is a Lagrange's undetermined multiplier, and

[Formula 25]

$$s^{(k)} = \sum_{j \neq k}^{K} S_{:j}$$

Through differentiation with respect to $S_{:k}$,

[Formula 26]

$$\frac{\partial J(S_{:k})}{\partial S_{:k}} = \frac{1}{\sigma^2}\left[X^{(k)\ T}C_{:k} + \ \|C_{:k}\|^2 S_{:k}\right] + \xi s^{(k)}$$

,

[Formula 27]

$$\frac{\partial J(S_{:k})}{\partial S_{:k}} = 0$$

.

By defining these formulas, an update formula for $S_{:k}$ is obtained as

[Formula 28]

$$S_{:k} \leftarrow \frac{-\sigma^2 \xi_k s^{(k)} + X^{(k)\ T} C_{:k}}{\|C_{:k}\|^2}$$

Regarding $\xi_k$ to apply a complete orthogonal condition,

[Formula 29]

$$s^{(k)\ T} S_{:k} = 0$$

on the basis this formula, $\xi_k$ can be estimated as

[Formula 30]

$$\xi_k = \frac{s^{(k)\ T} X^{(k)\ T} C_{:k}}{\sigma^2 s^{(k)\ T} s^{(k)}}$$

Regarding a soft orthogonal condition, a degree of orthogonality can be adjusted freely between complete orthogonality and no orthogonality constraint by multiplying $\xi_k$ by a hyper parameter w [0, 1]. A final update formula is

[Formula 31]

$$S_{:k} \leftarrow \frac{-\sigma^2 w \xi_k s^{(k)} + X^{(k)\,T} C_{:k}}{\|C_{:k}\|^2}$$

$$S_{:k} \leftarrow \frac{S_{:k}}{\|S_{:k}\|}$$

An update formula for C will be described. Extracting only a term $J(C_{:k})$ related to $C_{:k}$ from a negative log likelihood function is considered first.

[Formula 32]

$$J(C_{:k}) = \frac{1}{2\sigma^2}\left[-2S_{:k}^T X^{(k)} C_{:k} + \|C_{:k}\|^2\|S_{:k}\|^2\right] + \frac{1}{\lambda_k}\sum_{i=1}^{N} C_{i.}$$

$$\frac{\partial J(C_{:k})}{\partial C_{:k}} = \frac{1}{\sigma^2}\left[X^{(k)} S_{:k} + \|S_{:k}\|^2 C_{:k}\right] + \frac{1}{\lambda_k}$$

[Formula 33]

$$\|S_{:k}\|^2 = 1$$

By defining normalization in this way, on the basis of the following:

[Formula 34]

$$\frac{\partial J(C_{:k})}{\partial C_{:k}} = 0$$

,
the update formula is written as

[Formula 35]

$$C_{:k} \leftarrow X^{(k)} S_{:k} - \frac{\sigma^2}{\lambda_k}$$

A specific algorithm is as follows:

[Formula 36]

<u>Algorithm 1</u>: Pseudo-code of ARD-SO-NMF for TDMS

**Input**: Data $X \in \mathbb{R}^{N \times M}$, orthogonal constrain weight $w$, initial component number $K$, small positive number $a$, iteration number $L$, $threshold \in [0.9, 0.99]$

**Output**: optimized component number $K^*$, Cardinality matrix $C \in \mathbb{R}^{N \times K}$, base spectra $S \in \mathbb{R}^{M \times K}$, components importance $\lambda \in \mathbb{R}^K$

**//Initialization**

set $C, b$ by Eq. (1-2)

set $S$ by randomly selecting raw vector from $X$

**//Main loop**

**for** $l$ from 1 to $L$:

  **for** $k$ from 1 to $K$:

    update $S_{:k}$ by Eq. (6)

    $S_{:k} \leftarrow (S_{:k} + |S_{:k}|)/2$

  **end for**

  **for** $k$ from 1 to $K$:

    update $C_{:k}$ by Eq. (7)

    $C_{:k} \leftarrow (C_{:k} + |C_{:k}|)/2$

  **end for**

  **for** $k$ from 1 to $K - 1$:

    **for** $m$ from $k + 1$ to K:

      **//Merging C**

      **if** $S_{:k}^T S_{:m} > t$:

        $C_{:k} \leftarrow C_{:k} + C_{:m}$

        $C_{:m} \leftarrow 0$

      **end if**

      **//Merging S**

      **if** $C_{:k}^T C_{:m} > threshold \|C_{:k}\| \|C_{:m}\|$:

        $S_{:k} \leftarrow S_{:k} + \frac{\|C_{:m}\|_1}{\|C_{:k}\|_1} S_{:m}, \quad s = \|S_{:k}\|, \quad S_{:k} \leftarrow \frac{S_k}{\|S_{:k}\|}$

        $C_{:k} \leftarrow sC_{:k}, C_{:m} \leftarrow 0$

      **end if**

    **end for**

  **end for**

  update $\lambda$ by Eq. (4)

  update $\sigma^2$ by Eq. (3)

**end**

Referring back to Fig. 1, the base spectrum matrix and the data matrix are subjected to canonical correlation analysis to acquire a corrected intensity distribution matrix (step S105).

More specifically, a noise component in the intensity distribution matrix is extracted through analysis on canonical correlation between the base spectrum matrix and the data matrix and the intensity distribution matrix is corrected so as to reduce influence by the noise component, thereby acquiring the corrected intensity distribution matrix.

The NMF is low-rank approximation of a data matrix. Thus, even if the component k is not actually present in an i-th spectrum, $C_{ik} > 0$ is established in a case where assuming the presence of the component k provides better approximation in the sense of a least square. In many cases, such $C_{ik}$ is considerably small and often does not cause any problem in the NMF factorization.

In detecting a minor component, however, distinction is preferably made between $C_{jk} > 0$ actually present in tiny quantity in a j-th spectrum and $C_{ik} > 0$ as an NMF artifact, and 0 is preferably substituted into $C_{ik}$ as a ghost peak. The reason for this is that removing a spurious peak derived from the artifact from the NMF algorithm as one noise makes it possible to provide inference result with higher precision.

In the present step, canonical correlation analysis is employed to solve the foregoing issue. The present step is one of features of the present method and was named canonical correlation analysis (CCA) filter by the present inventors.

Conceptually, the CCA filter is to make sample-wise scanning to see whether each component in a base spectrum $S^T$ output from the NMF is actually contained in original data, and to delete the component from this spectrum of a sample if a similar peak pattern is not observed in the original data. If spectra generated from an L-th sample are stored from an 1-th row to an 1'-th row in the data matrix X, a submatrix obtained by extracting this part is written as $X_{\delta(L)}$. Specifically, $\delta(L) = (1, 1 + 1, ..., 1'-2, 1'-1)$.

The CCA is conventionally known as a technique of examining correlation between two-system data segments and is formulated as follows.

A matrix composed of N p-dimensional vectors is written as

[Formula 37]

$$Y \in \mathbb{R}^{p \times N}$$

.

A matrix composed of N q-dimensional vectors is written as

[Formula 38]

$$Z \in \mathbb{R}^{q \times n}$$

Then, the following formula is generated:

[Formula 39]

$$\rho = \frac{a^T V_{yz} b}{\sqrt{a^T V_{yy} a} \sqrt{b^T V_{zz} b}}$$

$$\max_{a,b} \rho$$

Here,

[Formula 40]

$$V_{YY} = YY^T/N, V_{ZZ} = ZZ^T/N, V_{YZ} = YZ^T/N$$

is established.

Specifically, a problem set to be solved by the CCA is to project Y and Z onto one axis and find a solution $(a^T, b^T)^T$ with which a correlation coefficient $\rho$ therebetween becomes maximum.

Here, $(a^T, b^T)^T$ and the correlation coefficient $\rho$ are given as a characteristic vector and a characteristic value relating to a generalized eigenvalue problem in the following formula, and all the characteristic vectors $(a^T, b^T)^T$ are written collectively as $(A^T, B^T)^T$. Then, the following formula is generated:

[Formula 41]

$$\begin{pmatrix} O & V_{yz} \\ V_{yz}^T & O \end{pmatrix} \begin{pmatrix} A \\ B \end{pmatrix} = \begin{pmatrix} V_{yy} & O \\ O & V_{zz} \end{pmatrix} \begin{pmatrix} A \\ B \end{pmatrix} \begin{pmatrix} \rho_1 & & \\ & \ddots & \\ & & \rho_{p+q} \end{pmatrix}, \rho_1 \geq \cdots \geq \rho_{p+q}$$

.

Here, it is assumed that the presence or absence of the base spectrum $S_{:k}$ of one component k in a sample L is to be judged. First, a pertinent partial data matrix $X_{\delta(L)}$ is extracted from the data matrix X. Meanwhile, $S^T$ is divided into two matrices including a matrix of a group of spectra $S_{sim}{}^T$ all similar to $S_{:k}{}^T$ and a matrix of a group of spectra $S_{out}{}^T$ largely different from $S_{:k}{}^T$.

[Formula 42]

$$Y = \begin{pmatrix} S_{out}^{\ T} \\ X_{\delta(L)} \end{pmatrix}$$

$$Z = \begin{pmatrix} S_{:k}^{\ T} \\ S_{sim}^{\ T} \end{pmatrix}$$

Then, the CCA is performed in this way. Here, all characteristic values satisfying $\rho > t_1$ are extracted with respect to a certain value $t \in [0,1]$. If a first component of a corresponding characteristic vector b satisfies $b_1 > t_2 b_{max}$ with respect to a maximum component of this vector, it is determined that the base spectrum $S_{:k}$ of the component k is contained in the sample L. If not, corresponding $C_{\delta(L)k}$ is determined to be 0.

Here, as $t_1$ and $t_2$ are hyper parameters and are strongly influenced by fitting precision of the NMF, specifically, by a threshold as an NMF hyper parameter, deliberate examination is required. Generally, $t_1 = 0.9$ threshold and $t_2 \in [0.1, 0.3]$ are found to produce favorable result from experience.

In the example given above, the CCA filter is applied to the NMF artifact (noise). However, the foregoing noise removal is applicable not only to the NMF artifact but also to removal of background or contamination.

Specifically, examples of noise removable by the CCA filter include: (1) an artifact from the NMF (so-called ghost peak); (2) a peak derived from a minor chemical material mixed from an environment around the device (background); and (3) a peak derived from a material contained in a sample and having a risk of distorting the substance of the sample if incorporated in analysis (contamination).

The sample set handled by the present method contains a background sample. Thus, by defining the following formula:

[Formula 43]

$$Y = \begin{pmatrix} S_{out}{}^T \\ X_{\delta(BG)} \end{pmatrix}$$

,

it becomes possible to judge whether the base spectrum $S_{:k}$ is contained in a background file. The presence of the base spectrum $S_{:k}$ therein means that the base spectrum $S_{:k}$ is system noise derived from an environment. Thus, this spectrum can be removed from the sample by defining $C_{:k} = 0$.

If the background sample further contains a contamination material, noise derived from the contamination can also be removed by following the same procedure as that described above. While the contamination material is not particularly limited, it may be a solvent used for preparation or loading of a sample, for example.

The present step may further include a step of correcting an intensity in an intensity distribution matrix.

For example, the DART-MS ionizes water or ammonia in the air primarily using helium ions, thereby ionizing a sample secondarily. For this reason, a peak intensity may be changed by an atmospheric condition such as a humidity. Furthermore, the mass of a sample to be loaded generally differs between samples and this also influences a peak intensity. Moreover, the ionization efficiency of each component contained in a sample generally differs between samples and this also influences a peak intensity.

By correcting the intensity in the intensity distribution matrix, it becomes possible to obtain more accurate quantitative result by alleviating these influences.

If all samples contain all the K types of components and if these samples include a calibration sample having a known composition, the intensity correction can be made by normalizing the intensity distribution matrix.

Normalizing the intensity distribution matrix means normalizing the matrix C for each sample, more specifically, means performing operation of adding all elements to a total of 1 for each sample.

Partitioning the matrix C sample-wise and writing the matrix C derived from the sample L as $C_{\delta(L)}$,

[Formula 44]

$$\widehat{C_{\delta(L)}} = \frac{C_{\delta(L)}}{\sum_{i,j}(C_{\delta(L)})_{ij}}, L \ for \ all \ samples$$

As information about a pyrolytic temperature is unnecessary in composition analysis,

[Formula 45]

$$\widehat{\overline{C}}_{\delta(L)}$$

is added in a vertical direction to obtain the following vector:

[Formula 46]

$$\widehat{\overline{c}}_L \in \mathbb{R}^K$$

This vector is used as a feature quantity vector of the sample L. Next, feature vectors of respective p single component samples measured as end members are aligned to form an end member matrix:

[Formula 47]

$$P \in \mathbb{R}^{K \times p}$$

A feature vector of a mixed sample can be written as

[Formula 48]

$$\widehat{\overline{c}}_L$$

as a linear sum of the feature vectors of the end members. Thus, using a coefficient vector thereof as follows:

[Formula 49]

$$r_L \in \mathbb{R}^p$$

,

the following formula can be defined:

[Formula 50]

$$\widehat{c_L} = Pr_L, subject\ to\ r_L \geq 0, \|r_L\|_1 = 1$$

.

As one calibration sample necessarily gives one or more pyrolysis samples, the number of components after the pyrolysis is written in a vertically-long matrix of a full column rank. For this reason, a solution is generally infeasible and an approximate solution in the sense of a least square can be obtained by quadratic programming.

In the mass spectrum acquired by the present method, ionization efficiency differs between compounds, pyrolysis efficiency also differs therebetween, and a compound quantity and a peak intensity are not proportionate to each other.

However, a peak intensity ratio including the ionization efficiency of the component k acquired by the NMF is already reflected in $S_{:k}^T$ and an intensity ratio between spectra is reflected in $C_{:k}$.

By the provision of such a hierarchical data configuration formed by the NMF, as long as consideration is given to a peak intensity ratio in an end member, it is possible to make more accurate quantitative composition analysis even without the need of determining unknown ionization efficiency.

Meanwhile, if the sample set does not include a calibration sample, at least part of (preferably, all of) the intensity distribution matrix is allocated using the product of the mass of the corresponding sample and an environmental variable. By doing so, it becomes possible to make more accurate quantitative analysis.

The environmental variable mentioned herein is a variable representing influence on ionization efficiency of each component during observation.

If a component has high ionization efficiency, a peak intensity is increased. If a component has low ionization efficiency, a peak intensity is reduced.

Regarding the mass of a sample, a peak intensity is generally increased with increase in the mass and a peak intensity is generally reduced with reduction in the mass.

If the sample set does not include a calibration sample, the matrix C is corrected as follows so as to make intensity information between samples held in the intensity distribution matrix available without normalizing the intensity distribution matrix:

[Formula 51]

$$\widetilde{C_{\delta(L)}} = \frac{C_{\delta(L)}}{m_L I_L}, L\ for\ all\ samples$$

Here, $m_L$ is the mass of the sample L, and $I_L$ is a peak intensity in (poly)dimethylsiloxane generated from an oil pump annexed to the DART-MS observed at 610 m/z in an entire temperature range. Furthermore, $I_L$ reflects a standby state during the measurement and a peak intensity actually differs by about three times between a rainy day and a sunny day. As a result of the correction,

[Formula 52]

$$\widetilde{c}_{\delta(L)}$$

is obtained and this is added in a vertical direction to obtain the following vector:

[Formula 53]

$$\widetilde{c_{\delta(L)}} \in \mathbb{R}^{K}$$

This vector is used as a feature vector of the sample L. Then, an end member matrix is formulated from a feature vector of an end member in the same way as follows:

[Formula 53]

$$P \in \mathbb{R}^{K \times p}$$

Then, on the basis of the following:

[Formula 55]

$$\widetilde{c_{\delta(L)}} = P r_L, subject\ to\ r_L \geq 0, \|r_L\|_1 = 1$$

,

$r_L$ is determined.

While $I_L$ has been described as being derived from poly(dimethylsiloxane) and having 610 m/z, it is not limited to that but is preferably derived from a compound having a molecular weight of 50-1500 contained in a predetermined quantity in an atmosphere during observation and/or is preferably derived from an organic low-molecular compound having a molecular weight of 50-500 contained in a predetermined quantity in each of all the samples.

Referring back to Fig. 1, the corrected intensity distribution matrix is partitioned into submatrices corresponding to respective ones of the samples in the sample set, and the submatrices are used as feature vectors to express the respective corresponding samples in vector space (step S106).

Furthermore, a K-1 dimensional simplex including all of these feature vectors (each corresponding to one sample) is defined, and K end members in the K-1 dimensional simplex are determined (step S107).

If the learning sample contains an end member and the learning sample is given a determination label (a label for determining that this learning sample is an "end member"), the end member is determined on the basis of the label.

If the learning sample contains an end member and the learning sample is not given a determination label, specifically, if the learning sample contains an end member but it is unknown which learning sample contains the end member, the end member is determined through vertex component analysis on a feature vector.

If the learning sample does not contain an end member, an end member is determined on the basis of an algorithm by which a vertex is defined in such a manner that the K-1 dimensional simplex has a minimum volume. If the K-1 dimensional simplex is a two-dimensional simplex (triangle), the minimum volume of the K-1 dimensional simplex means a minimum area thereof.

According to one aspect of the present method, even if the learning sample does not contain an end member, at least one feature vector of the learning sample is present in each region external to a hyperspherical body inscribed in the foregoing K-1 dimensional simplex (if K is equal to or greater than 3), making it possible to determine an end member precisely.

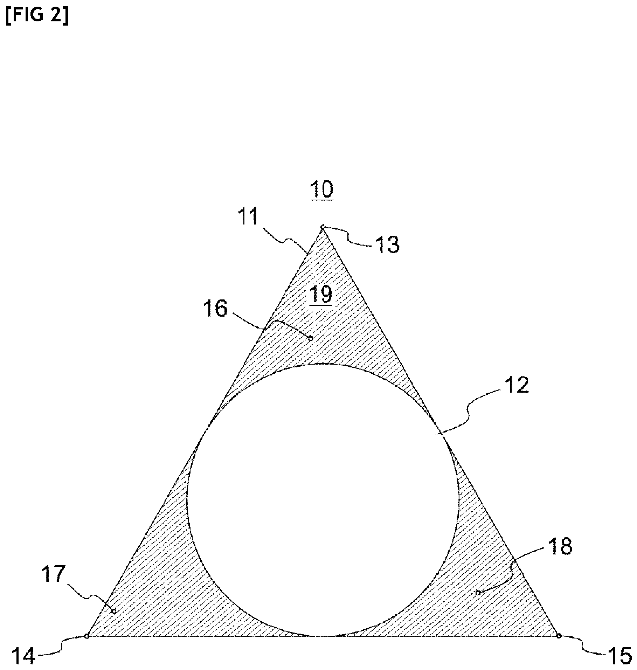

Fig. 2 is a conceptual view showing the K-1 dimensional simplex (two-dimensional simplex, a triangle) while K is 3. A K-1 dimensional simplex 10 is a triangle with vertexes defined by end members 13, 14, and 15 determined by learning samples 16, 17, and 18 respectively.

As shown in Fig. 2, if all the learning samples 16, 17, and 18 are present in a region 19 (hatched) external to a hypersphere 12 (in this case, a "circle") inscribed in the K-1 dimensional simplex, high-precision quantitative analysis result is obtained.

This was devised on the basis of Robust Volume Minimization-based Matrix Factorization for remote Sensing and Document Clustering, IEEE TRANSACTIONS ON SIGNAL PROCESSING, VOL. 64, NO. 23, DECEMBER 1, 2016.

There is no particular limitation on the algorithm by which a vertex is defined in such a manner that the K-1 dimensional simplex has a minimum volume but a publiclyknown algorithm is applicable. Examples of such an algorithm include a minimum volume constraint-NMF method, a minimum distance constraint-NMF method, a simplex identification via split augmented Lagrangian method, and a simplex volume minimization method.

Next, a Euclidean distance between each of K end members and a feature vector of an inference target sample is calculated to infer a content ratio of each component in the sample (step S108).

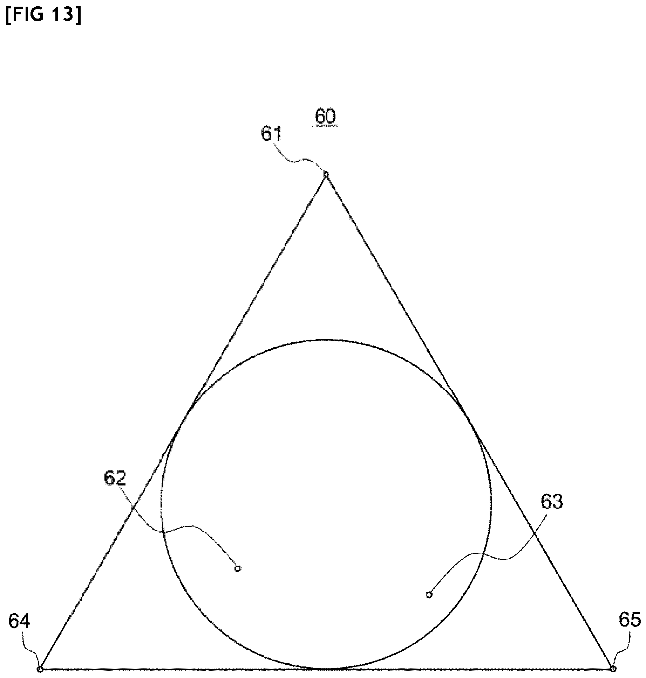

According to another aspect of the present method, if the learning sample contains at least one end member (if at least one learning sample is an end member), a feature vector of another learning sample may be present in a region inside a hyperspherical body inscribed in the K-1 dimensional simplex. Like Fig. 2, Fig. 13 is a conceptual view showing a K-1 dimensional simplex (two-dimensional simplex, a triangle) while K is 3. A K-1 dimensional simplex 60 is a triangle with vertexes defined by a learning sample 61 as an end member, and by end members 64 and 65 determined by different learning samples 62 and 63 respectively. A difference from Fig. 2 is that the learning samples 62 and 63 are present in a region inside a hypersphere inscribed in the K-1 dimensional simplex.

Even if at least one learning sample is an end member, a different learning sample may still be present on a hypersphere inscribed in the K-1 dimensional simplex or in a region external to the hypersphere. Specifically, in this case, the other learning sample may be present at any position.

In terms of achieving more excellent effect of the present invention, it is particularly preferable that the other learning sample contain 20% by mass or more, more preferably, 40% by mass or more of a component in an end member different from the learning sample as an end member (a component in a different end member).

According to the present method, even if a sample is a solid sample hard to dissolve in a solvent and hard to undergo conventional composition analysis, this sample is still available for analysis without particular preparatory process and can be subjected to accurate quantitative analysis. The present method achieves significant reduction in time required for obtaining result, particularly by being applied to quality control of a sample as a mixture of a plurality of components, investigation of a cause for failure, etc.

[Second Embodiment of Present Method]

A second embodiment of the present method includes a method of determining an end member (vertex) applied if the learning sample does not include an end member, specifically, if there is no learning sample at a vertex of the K-1 dimensional simplex in the step S107 in the first embodiment already described.

One feature of the method according to the present embodiment is that an end member is determined by second NMF process by which a matrix representing a fragment abundance of each sample is subjected to non-negative matrix factorization to be factorized into the product of a matrix representing the weight fractions of the K types of components in the sample and a matrix representing a fragment abundance of each simplex of the K types of components.

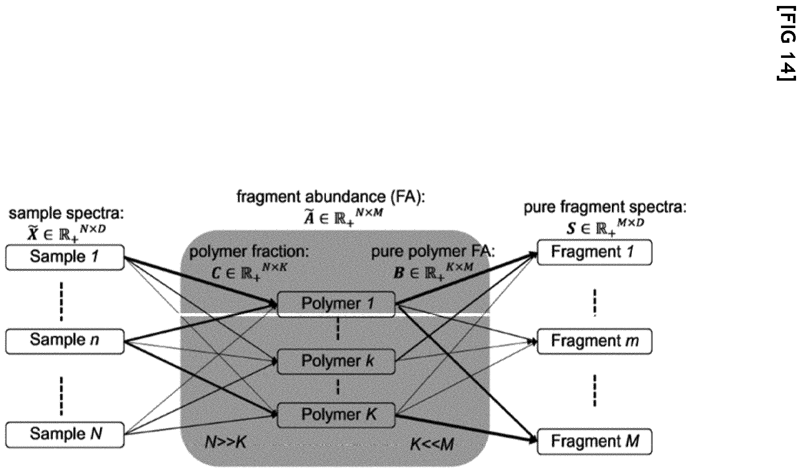

Fig. 14 is a schematic view for explaining a mechanism thereof.

While each sample is a mixture of the K types of components (this will be described as a mixture of "polymers"), what can actually be measured is an appropriate linear sum of spectra S of M types of fragment gases generated by pyrolysis of each polymer.

This feature according to the present embodiment is based on an idea that, by expressing a coefficient matrix thereof as

[Formula 56]

$$\tilde{A} \in \mathbb{R}_+^{N \times M}$$

[Formula 57]

$$\tilde{X} \approx \tilde{A} S$$

is formulated. Assuming that simplexes of the K polymers can be subjected to measurement and can be observed as

[Formula 58]

$$BS \in \mathbb{R}_+^{K \times D}$$

[Formula 59]

$$\tilde{A} \approx CB$$

a composition C can be obtained by performing the second NMF process in this way.

Here,

[Formula 60]

$$B \in \mathbb{R}_+^{K \times M}$$

is an abundance of M fragments generated from the K simplexes (polymers).

The following describes the details of a difference between the NMF process in step S104 (hereinafter also called "first NMF process") and the NMF process performed in determining an end member in step S107 (hereinafter also called "second NMF process") in the flow of Fig. 1, and others. In principle, steps, etc. not described below are the same as the corresponding steps in the first embodiment.

In the following description, symbols, etc. used in the description of the first embodiment will be replaced with those shown in the following table:

[Table 1]

| Term | Before replacement | After replacement |
|---|---|---|
| Data matrix | X:N spectra $\times$ M channels | (The number N of samples $\times$ the number of temperature zones) $\times$ D channel |
| | | This is defined as $X \in \mathbb{R}_+^{NT \times D}$ (meaning non-negative NT $\times$ D matrix) |
| The number of basis fragments | K | M |
| Intensity distribution matrix | $C \in \mathbb{R}_+^{N \times K}$ | $A \in \mathbb{R}_+^{NT \times M}$ |
| Base spectrum matrix | $S \in \mathbb{R}_+^{M \times K}$ | $S \in \mathbb{R}_+^{M \times D}$ |
| Characteristic vector | $(a^T, b^T)^T$ | $(u^*, v^*)$ |
| Matrix representation of characteristic vector | $\begin{pmatrix} A \\ B \end{pmatrix}$ | $\begin{pmatrix} U \\ V \end{pmatrix}$ |

Furthermore, in many cases, notations used in the following description follow a system generally employed in signal processing.

[Formula 61]

$$\mathbb{R}_+^{N \times M}, \quad \mathbb{R}^{N \times M}$$

This is a non-negative or real-number N $\times$ M matrix.

Regarding the following matrix

[Formula 62]

$$X \in \mathbb{R}_+^{N \times M},$$

[Formula 63]

$$X_{n:} \in \mathbb{R}_+^{1 \times M}, X_{:m} \in \mathbb{R}_+^{N \times 1}, X_{nm} \in \mathbb{R}_+$$

represents an n-th row vector, an m-th column vector, and an (n, m)-th element. Furthermore, $X^T$ is a transposed matrix of X, and

[Formula 64]

$$\|X\|_F$$

represents a Frobenius norm.

[Formula 65]

$$\|X_{n:}\|_1 \; , \; \|X_{n:}\|_2$$

represent the following in an n-th row of X:

[Formula 66]

$$\ell_1\text{-norm}, \quad \ell_2\text{-norm}$$

If X is a square matrix, Tr(X) represents a trace and diag(X) represents a diagonal matrix composed of a diagonal component. Furthermore, $1_N$ and $11_N$ represent an n-dimensional vector or an (N, N) matrix where all elements are 1. Moreover, $I_N$ represents an N-dimensional unit matrix.

(First NMF)

[Formula 67]

$$X \approx AS$$

Input: data matrix

[Formula 68]

$$X \in \mathbb{R}_+^{NT \times D}$$

Output: distribution matrix

[Formula 69]

$$A \in \mathbb{R}_+^{NT \times M}$$

A basis fragment spectrum

[Formula 70]

$$S \in \mathbb{R}_+^{M \times D}$$

Here, N is the number of samples, T is the number of temperature zone partitions, D is the number of channels, and M is the number of base spectra.

The first NMF was developed so as to achieve better conformity with data interpretation of MS (mass spectrometry) by adding the following three points as main changes to ARD-SO-NMF proposed by Shiga, et al.

1) Variance-covariance matrix of Gaussian noise for each channel

[Formula 71]

$$R \in \mathbb{R}^{D \times D}$$

is estimated on the basis of a natural isotopic peak.
2) Application of soft orthogonal constraint between basis fragment spectra.
3) Merging of fragment spectra having similar intensity distributions (expansion of merging condition)

Regarding 1), Gaussian noise of an independent equal variance (i. i. d) is assumed with a variance $\sigma^2$ for some time and then a variance-covariance matrix R is introduced in the middle. Assuming i. i. d in the noise, a probability generative model for the data matrix X can be written as

[Formula 72]

$$p(X|A, S, \sigma^2) = \prod_{i=1}^{NT} \prod_{m=1}^{M} \frac{1}{\sqrt{2\pi\sigma^2}} exp\left\{-\frac{(X_{im} - [AS]_{im})^2}{2\sigma^2}\right\}$$

As the number M of base spectra is unknown, automatic relevance determination (ARD) is introduced on the basis of sparseness of a distribution matrix A for allowing this number to be inferred automatically. First, an exponential distribution parametrized with $\lambda_m$ for each basis component is assumed as a prior distribution of A.
Specifically, with respect to the following:

[Formula 73]

$$\lambda = (\lambda_1, \ldots, \lambda_M)^T \in \mathbb{R}^M$$

,

[Formula 74]

$$p(A_{im}|\lambda_m) = \frac{1}{\lambda_m} \exp\left(-\frac{A_{im}}{\lambda_m}\right) s.t. \lambda_m > 0, for\ i = 1, \ldots, NT, m = 1, \ldots, M,$$

$$p(A|\lambda) = \prod_{i=1}^{NT} \prod_{m=1}^{M} p(A_{im}|\lambda_m),$$

.

An entire probability model can be written as

[Formula 75]

$$p(X, A, S) = p(X|A, S, \sigma^2)p(A|\lambda)p(S)p(\lambda|a, b),$$

.

Here, p(S) is a uniform distribution on a hypersphere as follows:

[Formula 76]

$$\|S_{n:}\|_2 = 1$$

.

Furthermore, $p(\lambda|a, b)$ is an inverse gamma distribution parametrized with (a, b), which is specifically

[Formula 77]

$$p(\lambda|a, b) = \prod_{m=1}^{M} p(\lambda_m|a, b) = \frac{b^a}{\Gamma(a)} \lambda_m^{-(a+1)} \exp\left(-\frac{b}{\lambda_m}\right) for\ m = 1, ..., M,$$

Here, a is a hyper parameter for adjusting sparseness and a = 1 + 10^{-16}, and b can empirically be estimated from an expected value E $(A_{im})$ of $A_{im}$ and is related with
[Formula 78]

$$E(A_{im}) = \frac{b}{a - 1} \qquad (1)$$

This further has a relation with $E(X_{id})$ as follows:

[Formula 79]

$$E(X_{id}) = \sum_{m=1}^{M} E(A_{im})E(S_{md}) = M \cdot E(A_{im})E(S_{md}),$$

By approximating $E(X_{id})$ with an average $\mu_x$ of X

[Formula 80]

$$\mu_X = M \frac{b}{(a-1)\sqrt{D}},$$

b is defined as follows:

[Formula 81]

$$b = \frac{\mu_X (a-1)\sqrt{D}}{M}, \qquad (2)$$

Thus, a negative log likelihood function can be written as

[Formula 82]

$$L(X, A, S, \lambda) = -\log[p(X|A,S)p(A|\lambda)p(\lambda|a,b)]$$

$$= \frac{1}{2\sigma^2} \|X - AS\|_F^2 + \frac{DNT}{2} \log 2\pi\sigma^2 + (NT + a + 1) \sum_{m=1}^{M} \log\lambda_m$$

$$+ \sum_{m=1}^{M} \frac{1}{\lambda_m} \left( b + \sum_{i=1}^{NT} A_{im} \right) + M(\log\Gamma(a) - a\log b).$$

This is a downward-convex function with respect to $\lambda$. Thus, an update formula for $\lambda$ is obtained as

[Formula 83]

$$\frac{\partial L}{\partial \lambda} \equiv 0$$

,

specifically, as
[Formula 84]

$$\lambda_m = \frac{b + \sum_{i=1}^{NT} A_{im}}{NT + a + 1}, for\ m = 1, ..., M \qquad (S3)$$

Derivation of the ARD-SO-NMF proposed by Shiga, et al. has completely be followed so far.
The assumption of the i. i. d Gaussian distribution of the noise does not apply to MS data. A larger signal is known to be likely to have larger noise. Then, a noise distribution is determined as

[Formula 85]

$$\mathbf{E} \in \mathbb{R}^{NT \times D}$$

as a residual component of linear regression resulting from the natural isotopic peak. Specifically,
[Formula 86]

$$\mathbf{E}_{:d} = \mathbf{X}_{:d} - \mathbf{M}^{(d)}\left(\mathbf{M}^{(d)T}\mathbf{M}^{(d)}\right)^{-1}\mathbf{M}^{(d)T}\mathbf{X}_{:d}, for\ d = 1, ..., D, \qquad (S4)$$

Here,

[Formula 87]

$$\mathbf{M}^{(d)} = \mathbf{X}_{:[d-30, d-20,\ d-10,\ d+10,\ d+20, d+30]}$$

is a channel [d-30, d-20, d-10, d+10, d+20, d+30] centered around the channel d and is an isotropic peak of $\pm 3$ m/z of the channel d (note that channel spacing is 0.1 m/z). The variance-covariance matrix of each channel, which is given as

[Formula 88]

$$R \in \mathbb{R}^{D \times D}$$

is determined as follows:

[Formula 89]

$$R = \frac{1}{NT} E^T E$$

Using this formula, the likelihood function is rewritten as

[Formula 90]

$$p(X|A, S, R) = \frac{1}{\sqrt{2\pi}^{DNT} \sqrt{|R|}^{NT}} \exp\{-Tr[(X - AS)R^{-1}(X - AS)^T]\}.$$

Note that R is a constant matrix. A total negative log likelihood function is defined as

[Formula 91]

$$L(X, A, S, \lambda) = -\log[p(X|A, S, R)p(A|\lambda)p(\lambda|a, b)]$$

$$= Tr[(X - AS)R^{-1}(X - AS)^T] + \frac{DNT}{2} log2\pi + \frac{NT}{2} log|R|$$

$$+ (NT + a + 1) \sum_{m=1}^{M} log\lambda_m + \sum_{m=1}^{M} \frac{1}{\lambda_m} \left( b + \sum_{i=1}^{NT} A_{im} \right)$$

$$+ M(log\Gamma(a) - alogb).$$

By substituting the update formula Eq. S3 for $\lambda$ and making simplification by omitting a constant term,

[Formula 92]

$$L(X, A, S, \lambda) = Tr[(X - AS)R^{-1}(X - AS)^T] + (NT + a + 1) \sum_{m=1}^{M} \log \lambda_m,$$

is obtained. This function is minimized with respect to A and S by using hierarchical alternating least square (HALS). For simplification, the following vector notation is used:

[Formula 93]

$$a_m \equiv A_{:m}, s_m \equiv S_{m:}^T, for \ m = 1, .., M.$$

According to the HALS, a residual X-AS is expressed as

[Formula 94]

$$X^{(m)} - a_m s_m^T \ (m = 1, ..., M)$$

Here,

[Formula 95]

$$X^{(m)} = X - AS + a_m s_m^T$$

Then, L(X, A, S, λ) can be written separately between components m as

[Formula 96]

$$L(X, a_m, s_m, \lambda_m(a_m))$$

$$= Tr\left[(X^{(m)} - a_m s_m^T)R^{-1}(X^{(m)} - a_m s_m^T)^T\right] + (NT + a + 1)\log \lambda_m,$$

$$for \ m = 1, ..., M.$$

Soft orthogonal constraint on S can be incorporated as a penalty term as follows into an objective function L:

[Formula 97]

$$W_O \xi_m s_m^T s^{(m)}$$

.

Here,

[Formula 98]

$$s^{(m)} \equiv \sum_{j \neq m}^{M} s_j$$

is established. This term represents non-orthogonality of an m-component and other components, and $\xi_m$ represents a Lagrange's undetermined multiplier applied if orthogonality is satisfied in a strict sense. This term is eased further using $w_o \in [0, 1]$. Thus, the objective function to be minimized is defined as

[Formula 99]

$$L(X, a_m, s_m, \lambda_m(a_m))$$

$$= Tr\left[\left(X^{(m)} - a_m s_m^T\right) R^{-1} \left(X^{(m)} - a_m s_m^T\right)^T\right] + (NT + a + 1)\log \lambda_m$$

$$+ w_O \xi_m s_m^T s^{(m)},$$

Slopes with respect to $a_m$ and $s_m$ are as follows:

[Formula 100]

$$\frac{\partial L}{\partial \boldsymbol{a}_m} = \left(\boldsymbol{a}_m \boldsymbol{s}_m^T - \boldsymbol{X}^{(m)}\right) \boldsymbol{R}^{-1} \boldsymbol{s}_m + \frac{1}{\lambda_m} \mathbf{1}_{NT},$$

$$\frac{\partial L}{\partial \boldsymbol{s}_m} = \boldsymbol{R}^{-1} \left(\boldsymbol{s}_m \boldsymbol{a}_m^T - \boldsymbol{X}^{(m)^T}\right) \boldsymbol{a}_m + w_O \, \xi_m \boldsymbol{s}^{(m)},$$

and by assuming these values as zero, update formulas are written as

[Formula 101]

$$\boldsymbol{a}_m = \frac{\boldsymbol{X}^{(m)} \boldsymbol{R}^{-1} \boldsymbol{s}_m - \frac{1}{\lambda_m} \mathbf{1}_{NT}}{\boldsymbol{s}_m^T \boldsymbol{R}^{-1} \boldsymbol{s}_m}, \qquad (S5)$$

$$\boldsymbol{s}_m = \boldsymbol{X}^{(m)^T} \boldsymbol{a}_m - w_O \, \xi_m \boldsymbol{R} \boldsymbol{s}^{(m)}, \qquad (S6)$$

As $s_m$ is normalized in response to each update, a constant coefficient was omitted. Non-negative constraint was satisfied by being projected onto a non-negative quadrant in response to each update. As a specific example, the projection can be realized as

[Formula 102]

$$\boldsymbol{a}_m \leftarrow \frac{\boldsymbol{a}_m + |\boldsymbol{a}_m|}{2},$$

where,

[Formula 103]

$$\left| \boldsymbol{a}_m \right|$$

means a vector assuming an absolute value for each element. By multiplying Eq. S6 by

[Formula 104]

$$\boldsymbol{s}^{(m)^T} \boldsymbol{R}$$

from the left side and applying the following strict orthogonal condition and $w_o = 1$:

[Formula 105]

$$\boldsymbol{s}^{(m)^T} \boldsymbol{s}_m = 0$$

,

, the undetermined multiplier $\xi_m$ is obtained as

[Formula 106]

$$-\boldsymbol{s}^{(m)^T} \boldsymbol{X}^{(m)^T} \boldsymbol{a}_k + \xi_m \boldsymbol{s}^{(m)^T} \boldsymbol{R} \boldsymbol{s}^{(m)} = 0,$$

$$\xi_m = \frac{\boldsymbol{s}^{(m)^T} \boldsymbol{X}^{(m)^T} \boldsymbol{a}_k}{\boldsymbol{s}^{(m)^T} \boldsymbol{R} \boldsymbol{s}^{(m)}} \qquad (S7)$$

Using the equations S1 to S7 given above, an algorithm 1 is suggested as follows:

[Formula 107]

<u>**Algorithm 1**: Pseudo-code of the first NMF</u>

**Input**: $\ell_1$-normalized data; $X \in \mathbb{R}_+^{NT \times D}$, orthogonal constraint weight; $w_O$, initial component number; $M$, iteration number; $itr$, margining threshold; $t \in [0.9, 0.99]$.

**Output**: spectra-wise fragment abundance matrix $A \in \mathbb{R}_+^{NT \times M}$, $M$-fragment spectra $S \in \mathbb{R}_+^{M \times D}$ with optimized $M$.

**Initialization**

initialize $A$ by Eq. S1

initialize $S$ by random selection of $M$-row vectors from $X$ and $\ell_2$-normalization.

calculate $b$ by Eq. S2

initialize $\lambda$ by Eq. S3

calculate $E$ by Eq. S4 and $R = \frac{1}{NT} E^T E$

**Repeat until convergence criteria are satisfied:**

**for** $m = 1, \ldots, M$:

    calculate $s^{(m)} \leftarrow \sum_{j \neq m}^{M} s_j$ and calculate $\xi_m$ by Eq. S7

    update $s_m$ by Eq. S6.

    project $s_m$ to the non-negative orthant

    $\ell_2$-normalize $s_m$

**for** $m = 1, \ldots, M$:

    update $a_m$ by Eq. S5

    project $a_m$ to the non-negative orthant

\# Merging very similar components

**for** $k = 1, \ldots, M - 1$:

    **for** $m = k, \ldots, M$:

        **if** $s_k^T s_m > t$:

            $a_k \leftarrow a_k + a_m, \ a_m \leftarrow 0$

        **if** $a_k^T a_m > t \|a_k\| \|a_m\|$:

            $s_k \leftarrow s_k + \frac{\|a_m\|_1}{\|a_k\|_1} s_m, \ \ s = \|s_k\|_2, \ \ s_k \leftarrow \frac{s_k}{s}$

            $a_k \leftarrow s a_k, a_m \leftarrow 0$

update $\lambda$ by Eq. S3

Here, each time A and S are updated, similar components are merged with each other. While merging of components having similar spectra with each other has been suggested by Shiga et al., components having similar intensity distributions are further subjected to the merging mentioned herein. By doing so, an isotopic peak, a fragment series ionized through addition of different ions, an oligomer peak series having different numbers of monomers, etc. can be merged into one component, making it possible to provide result with a higher degree of interpretability.

(Derivation of Canonical correlation analysis filter (CCA-filter))

Input: Output of first NMF

[Formula 108]

$$S \in \mathbb{R}_+{}^{M \times D}$$

a background spectrum:

[Formula 109]

$$X_{BG} \in \mathbb{R}_+{}^{T \times D}$$

Output: List of component numbers judged to be derived from background

The m-component obtained by the first NMF includes a component derived from background or a tramp material. As such a component might distort composition analysis result and is preferably removed from A or S accordingly. Assuming that an M'-component is judged to be a component derived from background by the CCA-filter,

[Formula 110]

$$A \in \mathbb{R}_+{}^{NT \times M}$$

and

[Formula 111]

$$S \in \mathbb{R}_+{}^{M \times D}$$

are defined as

[Formula 112]

$$A \in \mathbb{R}_+^{NT \times (M - M')}$$

and

[Formula 113]

$$S \in \mathbb{R}_+^{(M - M') \times D}$$

respectively. While the number of components after application of the CCA-filter is M - M' for simplification, M is still used consistently.

To apply the CCA-filter, a background spectrum

[Formula 114]

$$X_{BG} \in \mathbb{R}_+^{T \times D}$$

is required to be incorporated in the data set and required to be used together with a sample spectrum for performing the first NMF. If the presence of any tramp material can be expected, a spectrum measured for the tramp material can be used as $X_{BG}$. According to the CCA-filter, components m = 1, ..., M are checked one by one to see whether their respective spectra $S_{m:}$ are contained in $X_{BG}$.

A first step is to partition S composed of an M-spectrum and subjected to:

[Formula 115]

$$\ell_2\text{-normalized}$$

into a spectrum set similar to $S_{m:}$:

[Formula 116]

$$Y \in \mathbb{R}_+^{M_{sim} \times D}$$

and
into a spectrum set dissimilar to $S_{m:}$:

[Formula 117]

$$Z \in \mathbb{R}_+^{M_{dis} \times D}$$

This partitioning is performed in such a manner as to satisfy

[Formula 118]

$$S_{m:}Y_{m':}^{T} \geq t_1, for\ m' = 1, \dots, M_{sim},$$

$$S_{m:}Z_{m':}^{T} < t_1, for\ m' = 1, \dots, M_{dis},$$

Here, $t_1 \in [0, 1]$ is a certain threshold and $t_1 = 0.2$ was consistently used in the present invention. Furthermore, as $S_{m:}$ is always contained in Y, $S_{m:}$ is stored in a first column of Y. Z is combined with $X_{BG}$ as

[Formula 119]

$$Z \leftarrow \begin{pmatrix} Z \\ X_{BG} \end{pmatrix},$$

In order to obtain an average zero, Y and Z are defined as

[Formula 120]

$$\bar{Y} = Y\left(I_D - \frac{1}{D}11_D\right) \in \mathbb{R}^{M_{sim} \times D}, \quad\quad (S8)$$

$$\bar{Z} = Z\left(I_D - \frac{1}{D}11_D\right) \in \mathbb{R}^{(M_{dis}+T) \times D}, \quad\quad (S9)$$

These two spectrum sets are subjected to the CCA. The CCA is to generate a pair of spectra as similar as possible to each other through linear combination inside the two spectrum sets.

It is assumed that coefficients of linear combination of

[Formula 121]

$$\bar{Y}$$

and

[Formula 122]

$$\bar{Z}$$

are stored in a vector

[Formula 123]

$$u \in \mathbb{R}^{M_{sim}}$$

and in a vector

[Formula 124]

$$v \in \mathbb{R}^{M_{dis}+T}$$

Accordingly, the spectrum pair can be written as

[Formula 125]

$$y \equiv u^T \overline{Y} \in \mathbb{R}^{1 \times D}$$

and

[Formula 126]

$$z \equiv v^T \overline{Z} \in \mathbb{R}^{1 \times D}$$

Similarity therebetween is evaluated using the correlation coefficient ρ as

[Formula 127]

$$\rho = \frac{u^T V_{yz} v}{\sqrt{u^T V_{yy} u} \sqrt{v^T V_{zz} v}},$$

where

[Formula 128]

$$V_{YY} = \overline{Y}\overline{Y}^T / D, V_{ZZ} = \overline{Z}\overline{Z}^T / D, V_{YZ} = \overline{Y}\overline{Z}^T / D.$$

A problem setting by the CCA can be written as

[Formula 129]

$$(\boldsymbol{u}^*, \boldsymbol{v}^*) = arg\ max_{\boldsymbol{u},\boldsymbol{v}}\ \rho.$$

A solution thereof, which is written as

[Formula 130]

$$\begin{pmatrix} \boldsymbol{u} \\ \boldsymbol{v} \end{pmatrix} \in \mathbb{R}^{M_{sim} + M_{dis} + T}$$

,

is provided as a solution of a generalized eigenvalue problem as

[Formula 131]

$$\begin{pmatrix} \boldsymbol{O} & V_{yz} \\ V_{yz}^{T} & \boldsymbol{O} \end{pmatrix} \begin{pmatrix} U \\ V \end{pmatrix} = \begin{pmatrix} V_{yy} & \boldsymbol{O} \\ \boldsymbol{O} & V_{zz} \end{pmatrix} \begin{pmatrix} U \\ V \end{pmatrix} \begin{pmatrix} \rho_1 & & \\ & \ddots & \\ & & \rho_{M_{sim}+M_{dis}+T} \end{pmatrix}, \rho_1 \geq \cdots \geq \rho_{M_{sim}+M_{dis}+T}, (S10)$$

, where

[Formula 132]

$$\begin{pmatrix} U \\ V \end{pmatrix}$$

is a matrix obtained by aligning the following characteristic vectors as column vectors in descending order of characteristic value:

[Formula 133]

$$\begin{pmatrix} u* \\ v* \end{pmatrix}$$

Each characteristic value

[Formula 134]

$$(\rho_1, \ldots, \rho_{M_{sim}+M_{dis}+T})$$

is a coefficient of correlation between y and z generated through linear combination using corresponding (u*, v*) as a coefficient. Here, u* corresponding to every characteristic value satisfying $\rho > t_2$ is extracted. If a first element corresponding to a coefficient of $S_{m:}$ is a large component largely contributing to u*, specifically, if

[Formula 135]

$$\frac{|u_1|}{\|u^*\|_1} \geq t_3$$

,

the component m is judged to be a component derived from background and removed from a system. Here, $t_2 \in [0.9, 0.99]$ and $t_3 \in [0, 1]$. These processes are summarized as follows as an algorithm 2:

[Formula 136]

<u>**Algorithm 2**</u>: CCA-filter

**Input**: $\ell_1$-normalized basis-spectra of the 1stNMF output; $S \in \mathbb{R}_+^{M \times D}$, a background spectrum; $X_{BG} \in \mathbb{R}_+^{T \times D}$, thresholds: $t_1 \in [0,1], t_2 \in [0,1], t_3 \in [0,1]$, in this study $(t_1, t_2, t_3) = (0.2, 0.9, 0.5)$ is consistently used.

**Output**: a list of the components judged as background components.

**for** $m = 1, \dots, M$:

  **for** $m' = 1, \dots, M$:

    classify $S_{m':}$ into $Y$ if $S_{m:}S_{m':}{}^T \geq t_1$ else into $Z$.

  $Z \leftarrow \begin{pmatrix} Z \\ X_{BG} \end{pmatrix}$

  calculate $\overline{Y}$ and $\overline{Z}$ by Eq. S8 and Eq. S9

  calculate $V_{YY} = \overline{Y}\overline{Y}^T / D, V_{ZZ} = \overline{Z}\overline{Z}^T / D, V_{YZ} = \overline{Y}\overline{Z}^T / D$

  obtain $U^* = (u^*_1, \dots, u^*_{M_{sim} + M_{dis} + T})$ and $(\rho_1, \dots, \rho_{M_{sim} + M_{dis} + T})$ by solving Eq. S10

  find $Q$ such that $\rho_Q \geq t_2$ and $\rho_{Q+1} < t_2$

  **for** $q = 1, \dots, Q$:

    **if** $\dfrac{|U^*_{1q}|}{\|U^*_{:q}\|_1} \geq t_3$, add $m$ in the background-component list

  **return** the background-component list

After the background component is identified, a corresponding column vector in A and a corresponding row vector in S are deleted to be removed from the system.

After removal of the M'-component derived from background, the following is output from the CCA-filter:

[Formula 137]

$$A \in \mathbb{R}_+^{NT \times (M - M')}$$

(This is expressed as follows while M - M' is replaced with M for simplification:)

[Formula 138]

$$A \in \mathbb{R}_+^{NT \times M}$$

This is used for correcting an allocation intensity on the basis of a sample quantity and an internal reference peak. Then,

[Formula 139]

$$a_n = vec(A^{(n)})$$

,

which is a one-dimensional vector generated from a submatrix section $A^{(n)}$ of A related to a sample n, may be used as a feature vector of the sample n and may be input in the second NMF. Meanwhile, as an M-fragment temperature distribution is unnecessary information for composition analysis, all temperature zones may be added for each sample so that M-fragment abundance (FA) may be obtained for each sample, and the following representing this abundance may be used as input:

[Formula 140]

$$\tilde{A} \in \mathbb{R}_+^{N \times M}$$

.

Here, for simplification, the second NMF is performed with respect to the following:

[Formula 141]

$$\tilde{A}$$

.

In the following section, fitting by non-negative least square (NNLS) is performed frequently. Using an optimum non-negative coefficient as

[Formula 142]

$$x \in \mathbb{R}_+^{n}$$

and through column vector linear combination of a constant matrix as

[Formula 143]

$$\Phi \in \mathbb{R}^{m \times n}$$

,

the problem mentioned herein is to approximate:

[Formula 144]

$$y \in \mathbb{R}^m$$

.

and is determined by the following:

[Formula 145]

$$x^* = \underset{x}{\operatorname{argmin}} \|y - \Phi x\|^2, s.t. \, x \geq 0,$$

While this can be solved by a large number of optimization techniques including alternating direction multiplier methods (ADMM) (1), ADMM-NNLS developed by Fu. et al. is used herein to solve this problem and a solution is represented as

[Formula 146]

$$x^* = NNLS(y, \Phi)$$

A non-negative coefficient vector $x_l^*(l = 1, ..., L)$ corresponding to an approximate vector set $Y = [y_1, ..., Y_L]$ can be calculated individually and is written as

[Formula 147]

$$x_l^* = NNLS(Y_{:l}, \Phi) \; for \; l = 1, .., L,$$

or in a matrix form, as

[Formula 148]

$$X^* = NNLS(Y, \Phi)$$

Here,

[Formula 149]

$$X^* = [x_1^*, \dots, x_L^*]$$

A similar problem, which is a problem with a restraint condition that a total sum of coefficient vectors will be 1, is called fully constrained least square (FCLS), can also be solved using ADMM, and is written as

[Formula 150]

$$x_l^* = FCLS(Y_{:l}, \Phi) \ for \ l = 1, \dots, L,$$

or in a matrix notation, as

[Formula 151]

$$X^* = FCLS(Y, \Phi)$$

(Second NMF)

Input: The following representing FA for each sample:

$$\tilde{A} \in \mathbb{R}_+^{N \times M}$$

,

a spectrum of each basis M-fragment:

[Formula 153]

$$S \in \mathbb{R}_+^{M \times D}$$

, and the number K of polymer components.

Output: Polymer weight fraction for each sample:

[Formula 154]

$$C \in \mathbb{R}_+^{N \times K}$$

, and FA for each unit weight K-base polymer:

[Formula 155]

$$B \in \mathbb{R}_+^{K \times M}$$

Second NMF:

[Formula 156]

$$\widetilde{A} \approx CB$$

is evaluates in terms of its approximate residual as follows using Riemannian metrics:

[Formula 157]

$$D_G\left(\widetilde{A} \middle| CB\right)$$

Specifically,

[Formula 158]

$$D_G(\widetilde{A}|CB) = Tr\left[(\widetilde{A} - CB)G(\widetilde{A} - CB)^T\right],$$

where

[Formula 159]

$$G = SS^T \in \mathbb{R}_+^{M \times M}$$

By obtaining a lower triangular matrix as follows through Cholesky factorization G = LL$^T$

[Formula 160]

$$L \in \mathbb{R}^{M \times M}$$

,

the following formula can be written:

[Formula 161]

$$D_G(\widetilde{A}|CB) = Tr\left[(\widetilde{A} - CB)LL^T(\widetilde{A} - CB)^T\right] = Tr\left[(\widehat{A} - C\widehat{B})(\widehat{A} - C\widehat{B})^T\right] = \|\widehat{A} - C\widehat{B}\|_F^2$$

Here,

[Formula 162]

$$\widehat{A} = \widetilde{A}L \in \mathbb{R}^{N \times M}, \widehat{B} = BL \in \mathbb{R}^{K \times M}$$

, which is equivalent to matrix factorization as follows:

[Formula 163]

$$\widehat{A} \approx C\widehat{B}$$

.

Defining a row vector as

[Formula 164]

$$\widehat{B}$$

,

a volume term of a simplex spanned by this row vector is written as

[Formula 165]

$$vol\left(\widehat{B}\right), \widehat{B}$$

,

and a non-orthogonality term between such row vectors is written as

[Formula 166]

$$nonorth\left(\widehat{B}\right)$$

Then, using $\alpha > 0$ and $\beta \in [0, 1]$ as weights,
[Formula 167]

$$(C^*, \widehat{B}^*) = \arg\min_{C, \widehat{B}} \frac{1}{2}\left\|\widehat{A} - C\widehat{B}\right\|_F^2 + \frac{\alpha}{2}vol\left(\widehat{B}\right) + \frac{\beta}{2}nonorth\left(\widehat{B}\right), \qquad (S11)$$

$$s.t. \widehat{B} = BL, B \geq 0, C \geq 0, C\mathbf{1}_K = \mathbf{1}_N.$$

On the basis of this formula,

[Formula 168]

$$\left( C^{*}, \widehat{B}^{*} \right)$$

is determined. Here, in order to provide robustness to an outlier (3), introduction of the following weighting matrix is suggested by Fu et al.:

[Formula 169]

$$w_n = \frac{p}{2} \left( \left\| \widehat{A}_{n:} - C_{n:}\widehat{B} \right\|^2 + \varepsilon \right)^{\frac{p-2}{2}}$$

$$W = diag(w_1, \dots w_N)$$

Here, $p \in (0, 2]$, and $\varepsilon$ is a small regularization parameter. According to the present invention, $p = 1.5$ and $\varepsilon = 10^{-8}$ were consistently used. An optimization program is written as

[Formula 170]

$$\min_{C,\widehat{B}} \frac{1}{2} Tr\left[ W(\widehat{A} - C\widehat{B})(\widehat{A} - C\widehat{B})^T \right] + \frac{\alpha}{2} vol(\widehat{B}) + \frac{\beta}{2} nonorth(\widehat{B}),$$

$$s.t. \widehat{B} = BL, B \geq 0, C \geq 0, C1_K = 1_N.$$

Block coordinate descent (BCD) theory is employed to alternately optimize C and

[Formula 171]

$$\widehat{B}$$

Here, it is assumed that optimization is performed t times to obtain

[Formula 172]

$$\left(\boldsymbol{C}^{(t)}, \widehat{\boldsymbol{B}}^{(t)}\right)$$

Using vertex component analysis (VCA) (4) for initialization, K row vectors approximate to a K-vertex of a simplex are selected from N-row vectors of

[Formula 173]

$$\widehat{\boldsymbol{A}}$$

.

Then, the selected vectors are defined as

[Formula 174]

$$\widehat{\boldsymbol{B}}^{(0)}$$

Regarding update of C based on the following:

[Formula 175]

$$\widehat{\boldsymbol{B}}^{(t)}$$

,

the update can be done simply by
[Formula 176]

$$\boldsymbol{C}^{(t+1)^{T}} = FCLS\left(\widehat{\boldsymbol{A}}^{T}, \widehat{\boldsymbol{B}}^{(t)^{T}}\right). \tag{S13}$$

Then, update based on $C^{(t)}$ from

[Formula 177]

$$\widehat{\boldsymbol{B}}^{(t)}$$

59

to

[Formula 178]

$$\widehat{\boldsymbol{B}}^{(t+1)}$$

will be considered. Here,

[Formula 179]

$$nonorth(\widehat{\boldsymbol{B}}) = Tr\left(\Lambda(\widehat{\boldsymbol{B}}\widehat{\boldsymbol{B}}^T - diag(\widehat{\boldsymbol{B}}\widehat{\boldsymbol{B}}^T))\right),$$

and

[Formula 180]

$$\Lambda \in \mathbb{R}^{K \times K}$$

is an undetermined multiplier. Furthermore,

[Formula 181]

$$vol(\widehat{\boldsymbol{B}}) = \log|\det(\widehat{\boldsymbol{B}}\widehat{\boldsymbol{B}}^T + \tau I_K)| = \log|\det(\boldsymbol{H})|,$$

where

[Formula 182]

$$\boldsymbol{H}(\widehat{\boldsymbol{B}}) = \widehat{\boldsymbol{B}}\widehat{\boldsymbol{B}}^T + \tau I_K, \tau$$

is a small regularization parameter, and $\tau = 10^{-8}$ was consistently used. Here,

[Formula 183]

$$vol\left(\widehat{\boldsymbol{B}}\right)$$

as a Majorizer function is introduced. Using a tangent inequality and on the basis of the following at previous timing:

[Formula 184]

$$\boldsymbol{H}^{(t)} = \boldsymbol{H}\left(\widehat{\boldsymbol{B}}^{(t)}\right)$$

,

[Formula 185]

$$\log|\det(\boldsymbol{H})| \leq \log\left|\det\left(\boldsymbol{H}^{(t)}\right)\right| + Tr\left[\left(\nabla_{\boldsymbol{H}^{(t)}} \log|\det(\boldsymbol{H})|\right)^{T}\left(\boldsymbol{H} - \boldsymbol{H}^{(t)}\right)\right]$$

is established. Here,

[Formula 186]

$$\nabla_{\boldsymbol{H}^{(t)}} \log|\det(\boldsymbol{H})|$$

is a slope at $H^{(t)}$ in the following:

[Formula 187]

$$\log|\det(\boldsymbol{H})|$$

Using

[Formula 188]

$$\nabla_{\boldsymbol{H}^{(t)}} \log|\det(\boldsymbol{H})| = \boldsymbol{H}^{(t)^{-T}},$$

[Formula 189]

$$vol(\widehat{\boldsymbol{B}}) = \log|\det(\boldsymbol{H})| \le Tr\left[\boldsymbol{H}^{(t)^{-1}}\boldsymbol{H}\right] + const = Tr\left[\boldsymbol{F}^{(t)}\widehat{\boldsymbol{B}}\widehat{\boldsymbol{B}}^{T}\right] + const,$$

where

[Formula 190]

$$\boldsymbol{F}^{(t)} = \boldsymbol{H}^{(t)^{-1}},$$

and const is a constant term. Thus, by replacing

[Formula 191]

$$vol\left(\widehat{\boldsymbol{B}}\right)$$

with

[Formula 192]

$$Tr\left[\boldsymbol{F}^{(t)}\widehat{\boldsymbol{B}}\widehat{\boldsymbol{B}}^{T}\right].$$

all penalty terms are written collectively as

[Formula 193]

$$\frac{\alpha}{2}vol(\widehat{B}) + \frac{\beta}{2}nonorth(\widehat{B}) \leq \frac{\alpha}{2}Tr(F^{(t)}\widehat{B}\widehat{B}^T) + \frac{\beta}{2}Tr(\Lambda^{(t)}\widehat{B}\widehat{B}^T) + const$$

$$= \frac{1}{2}Tr(V\widehat{B}\widehat{B}^T) + const,$$

where

[Formula 194]

$$V = \alpha F^{(t)} + \beta \Lambda^{(t)}.$$

Accordingly, update of

[Formula 195]

$$\widehat{B}^{(t+1)}$$

can be obtained by solving the following:
[Formula 196]

$$\widehat{B}^{(t+1)} = \arg\min_{\widehat{B}} \frac{1}{2}Tr\left[W(\widehat{A} - C^{(t)}\widehat{B})(\widehat{A} - C^{(t)}\widehat{B})^T\right] + \frac{1}{2}Tr(V\widehat{B}\widehat{B}^T) \qquad (S14)$$

$$s.t.\,\widehat{B} = BL, B \geq 0.$$

In order to organize complicated constraint conditions,

a constraint

[Formula 197]

$$\widehat{B} = BL$$

is incorporated into an objective function using a Lagrange's undetermined multiplier as follows:

[Formula 198]

$$Z \in \mathbb{R}^{K \times M}$$

,

thereby solving this problem in the framework of ADMM.

[Formula 199]

$$\left(B^{(t+1)}, \widehat{B}^{(t+1)}\right) = \arg\min_{B, \widehat{B}} \max_{Z} \left\{ f(\widehat{B}) + Tr\left[Z^T(\widehat{B} - BL)\right] - \frac{1}{2\mu} \|Z - Z'\|_F^2 \right\},$$

$$s.t. B \geq 0,$$

where

[Formula 200]

$$f(\widehat{B}) \equiv \frac{1}{2} Tr\left[W(\widehat{A} - C^{(t)}\widehat{B})(\widehat{A} - C^{(t)}\widehat{B})^T\right] + \frac{1}{2} Tr(V\widehat{B}\widehat{B}^T)$$

,

and $\mu$ is a hyper parameter for ADMM (here, $\mu$ = 1 was consistently used). Furthermore, Z' represents Z at previous timing.

If
[Formula 201]

$$Z = Z' + \mu(\widehat{B} - BL), \qquad\qquad (S15)$$

,

, the objective function provides the following maximized with respect to Z:

[Formula 202]

$$g(B, \widehat{B}; Z) \equiv f(\widehat{B}) + \frac{\mu}{2} \left\| \widehat{B} - BL + \frac{1}{\mu} Z \right\|_F^2, B \geq 0$$

.

This may be optimized with respect to

[Formula 203]

$$\left(B, \widehat{B}\right)$$

.

[Formula 204]

$$\left(B, \widehat{B}, Z\right)$$

was optimized cyclically and an algorithm was summarized as Algorithm 3.

[Formula 205]

**Algorithm3: ADMM for solving the optimization of Eq. S14**

**input:** $B^{(t)}, \widehat{B}^{(t)}$, hyperparameter $\mu$, function $g(B, \widehat{B})$

**output:** $B^{(t+1)}, \widehat{B}^{(t+1)}$

**initialize:** $q = 0, B_q \leftarrow B^{(t)}, \widehat{B}_q \leftarrow \widehat{B}^{(t)}, Z_q \leftarrow 0$

**repeat until convergence:**

$$\widehat{B}_{q+1} = \underset{\widehat{B}}{\operatorname{argmin}}\, g(\widehat{B}; B_q, Z_q), \text{ solved by Eq. S16}$$

$$B_{q+1} = \underset{B \geq 0}{\operatorname{argmin}}\, g(B; \widehat{B}_{q+1}, Z_q), \text{ solved by Eq. S17}$$

$$Z_{q+1} = Z_q + \mu(\widehat{B}_{q+1} - B_{q+1}L)$$

$$q \leftarrow q + 1$$

$$\text{if } \left\|\widehat{B}_q - B_q L\right\|_F^2 < 10^{-6}:$$

**end**

$$B^{(t+1)} \leftarrow B_q, \widehat{B}^{(t+1)} \leftarrow \widehat{B}_q.$$

Objective function as

[Formula 206]

$$g\left(\widehat{B}; B_q, Z_q\right)$$

is optimization of a quadratic function without restraint condition with respect to

[Formula 207]

$$\widehat{B}$$

,

it is minimized by the following:
[Formula 208]

$$\frac{\partial g}{\partial \widehat{B}} = \left(C^{(t)^T} W C^{(t+1)} + V + \mu I_K\right)\widehat{B} - C^{(t)^T} W\widehat{A} - B_q L + \frac{1}{\mu}Z \equiv 0$$

$$\widehat{B}_{q+1} = \left(C^{(t)^T} W C^{(t)} + V + \mu I_K\right)^{-1}\left(C^{(t)^T} W\widehat{A} + B_q L - \frac{1}{\mu}Z\right), \qquad (S16)$$

Furthermore,

[Formula 209]

$$g\left(B; \widehat{B}_{q+1}, Z_q\right)$$

can be solved by NNLS and is updated as
[Formula 210]

$$B_{q+1}{}^T = NNLS\left(\left(\widehat{B}_{q+1} + \frac{1}{\mu}Z\right)^T, L^T\right), \qquad (S17)$$

As a result of the foregoing, the following update formula for solving the original problem Eq. S 11 was obtained:

[Formula 211]

$$\left(\widehat{B}, B, C\right)$$

Finally, update of $V = \alpha F^{(t)} + \beta \Lambda^{(t)}$ is considered. Regarding $F^{(t)}$, it can be determined easily as
[Formula 212]

$$F^{(t)} = \left(\widehat{B}^{(t)}\widehat{B}^{(t)^T} + \tau I_K\right)^{-1}, \qquad (S18)$$

Regarding $\Lambda^{(t)}$, by combining strict orthogonal conditions, which are specifically

[Formula 213]

$$\widehat{B}\widehat{B}^T = diag\left(\widehat{B}\widehat{B}^T\right) \equiv D, \ \beta = 1$$

and
[Formula 214]

$$\frac{\partial f\left(\widehat{B}; C^{(t)}\right)}{\partial \widehat{B}} = C^{(t)^T}W\left(C^{(t)}\widehat{B} - \widehat{A}\right) + \left(\alpha F^{(t)} + \Lambda^{(t)}\right)\widehat{B} \equiv 0. \qquad (S19)$$

, and by multiplying Eq. S19 by the following from the right side:

[Formula 215]

$$\widehat{B}^T$$

, $\Lambda^{(t)}$ can be obtained as
[Formula 216]

$$C^{(t)^T}W\left(C^{(t)}D - \widehat{A}\widehat{B}^T\right) + \left(\alpha F^{(t)} + \Lambda^{(t)}\right)D \equiv 0,$$
$$\Lambda^{(t)} = C^{(t)^T}W\left(\widehat{A}\widehat{B}^T D^{-1} - C^{(t)}\right) - \alpha F^{(t)}. \qquad (S20)$$

Using Eq. S18 and Eq. S20,
[Formula 217]

$$V = \alpha(1 - \beta)\left(\widehat{B}^{(t)}\widehat{B}^{(t)^T} + \tau I_K\right)^{-1} + \beta C^{(t)^T}W\left(\widehat{A}\widehat{B}^T D^{-1} - C^{(t)}\right). \qquad (S21)$$

is given. Using the foregoing, an algorithm for solving the problem Eq. S11 is obtained as follows:

[Formula 218]

**Algorithm 4: Pseudo-code for the second NMF (solving optimization Eq. S11)**

**Input**: output of the first NMF (sample-wise FA; $\widetilde{A} \in \mathbb{R}_+^{N \times M}$, basis-fragment spectra; $S \in \mathbb{R}_+^{M \times D}$), basis-polymer number; $K$, weights for penalty terms; $(\alpha, \beta)$, weight for outliers; $p$

**Output**: polymer weight-fraction; $C \in \mathbb{R}_+^{N \times K}$, FAs of unit-weight basis-polymers $B \in \mathbb{R}_+^{K \times M}$

**Initialization**

calculate $L$ via Cholesky decomposition of $SS^T$

set $\widehat{A} = \widetilde{A}L$

initialize $\widehat{B}^{(0)}$ by selecting $K$-rows of $\widehat{A}$ via VCA algorithm

set $B^{(0)} = \widehat{B}^{(0)}L^{-1}$

initialize $C^{(0)}$ by $C^{(0)^T} = FCLS(\widehat{A}^T, \widehat{B}^{(0)^T})$

initialize $W$ by Eq. S12

initialize $V$ based on $B^{(0)}$ and $C^{(0)}$ by Eq. S21

**Repeat until criteria for convergence satisfied:**

   update $\widehat{B}, B$ by algorithm 3

   update $C$ by Eq. S13

   update $W, V$ by Eq. S12 and S21, respectively

(Projection of Test Data onto Hyperplane Spanned by S and B)

After S and B are inferred from the data set, the following data not having been used for the inference of S and B (here, called test data) is projected onto a hyperplane spanned by S and B by a method described herein:

[Formula 219]

$$X_{test} \in \mathbb{R}_+^{T \times D}$$

First, by the projection onto an S-hyperplane,

[Formula 220]

$$A_{test} \in \mathbb{R}_+{}^{T \times M}$$

is obtained. Specifically, a total sum is determined with respect to temperature zones

[Formula 221]

$$A_{test}{}^{T} = NNLS\left(R^{-\frac{1}{2}}X_{test}{}^{T}, R^{-\frac{1}{2}}S^{T}\right),$$

which is converted to

[Formula 222]

$$\tilde{A}_{test} \in \mathbb{R}_+{}^{1 \times M}$$

Then, by performing projection onto a B-hyperplane and normalization,

[Formula 223]

$$C_{test} \in \mathbb{R}_+{}^{1 \times K}$$

is obtained. Specifically,

[Formula 224]

$$C_{test}{}^T = NNLS\left(L^T \tilde{A}_{test}{}^T, L^T B^T\right),$$

$$C_{test} \leftarrow \frac{C_{test}}{\|C_{test}\|_1}.$$

The data set and hyper parameters are as follows:

[Table 2]

| Summary of dataset and hyperparameters. | | | | | | | | | |
|---|---|---|---|---|---|---|---|---|---|
| Sample number $N$ | Temp. range (°C) | First NMF | | | | | Second NMF | | |
| | | $w_o$ | Merging thres. | Initial $M$ | Prior | iteration | $K$ | $\alpha = \beta$ | $p$ |
| | Mass range(m/z) | | | | | | | | |
| 24 | [50, 450] [50, 1000] | 0.2 | 0.99 | 30 | Exp | 3000 | 3 | 0.1 | 1.5 |

Cited literatures are as follows:

[Table 3]

1.  S. Boyd, N. Parikh, E. Chu, B. Peleato, J. Eckstein, Distributed optimization and statistical learning via the alternating direction method of multipliers. Found. Trends Mach. Learn. 3, 1-122 (2010).
2.  X. Fu, W. Ma, K. Huang, N. D. Sidiropoulos, ROBUST VOLUME MINIMIZATION-BASED MATRIX FACTORIZATION VIA ALTERNATING OPTIMIZATION Department of ECE , University of Minnesota , Minneapolis , MN , USA Department of EE , The Chinese University of Hong Kong , Shatin , N . T ., Hong Kong. Icassp 2016. MI, 2534-2538 (2016).
3.  X. Fu, K. Huang, B. Yang, W. K. Ma, N. D. Sidiropoulos, Robust Volume Minimization-Based Matrix Factorization for Remote Sensing and Document Clustering. IEEE Trans. Signal Process. 64, 6254-6268 (2016).
4.  J. M. B. Dias, J. M. P. Nascimento, Vertex component analysis: A geometric-based approach to unmix hyperspectral data. Signal Image Process. Remote Sens. 43, 415-439 (2006).

(Composition Inference Device)

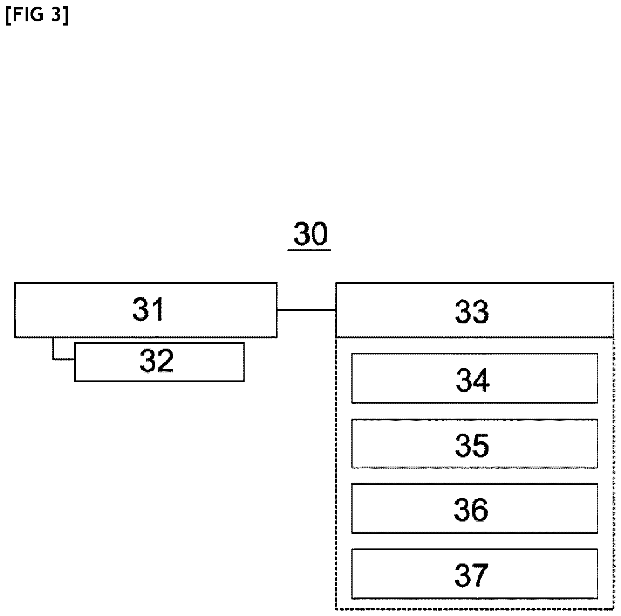

A composition inference device according to an embodiment of the present invention will be described next by referring to the drawings. Fig. 3 is a hardware configuration view of the composition inference device according to the embodiment of the present invention.

A composition inference device 30 includes a mass spectrometer 31 and an information processing device 33. The mass spectrometer 31 includes a pressure-reducing pump 32. The information processing device 33 includes a processor 34, a storage device 35, a display device 36, and an input device 37. The mass spectrometer 31 and the information processing device 33 are configured in such a manner that data can be transferred therebetween.

Examples of the processor 34 include a microprocessor, a processor core, a multiprocessor, an ASIC (application-specific integrated circuit), an FPGA (field programmable gate array), GPGPUs (general-purpose computing on graphics processing units), and the like.

The storage device 35 has the function of storing various types of programs and data in a transitory manner and/or a non-transitory manner, and provides a work area for the processor 34.

Examples of the storage device 35 include a ROM (read only memory), a RAM (random access memory), an HDD (hard disk drive), a flash memory, and an SSD (solid state drive).

The input device 37 can accept various types of information and can accept input of a command to the composition inference device 30. Examples of the input device 37 may include a keyboard, a mouse, a scanner, and a touch panel.

The display device 36 can display a status of the composition inference device 30, and progress of analysis, composition inference result, etc. by the composition inference device 30. Examples of the display device 36 may include a liquid crystal display and an organic EL (electro luminescence) display.

The display device 36 may be configured as a device integrated with the input device 37. In this case, the display device 36 may be configured as a touch panel display and may be configured to provide a GUI (graphical user interface).

The information processing device 33, which includes the processor 34, the storage device 35, the input device 37, and the display device 36 allowing communication of data therebetween through a data bus, is typically a computer.

The mass spectrometer 31 includes the pressure-reducing pump 32, and is typically a mass spectrometer including a DART ion source, a sample heater, and a timeof-flight type mass spectrometer instrument. Both the ion source and the mass spectrometer instrument of the mass spectrometer 31 are non-restrictive examples and the configuration of the mass spectrometer provided to the present composition inference device is not limited to that described above.

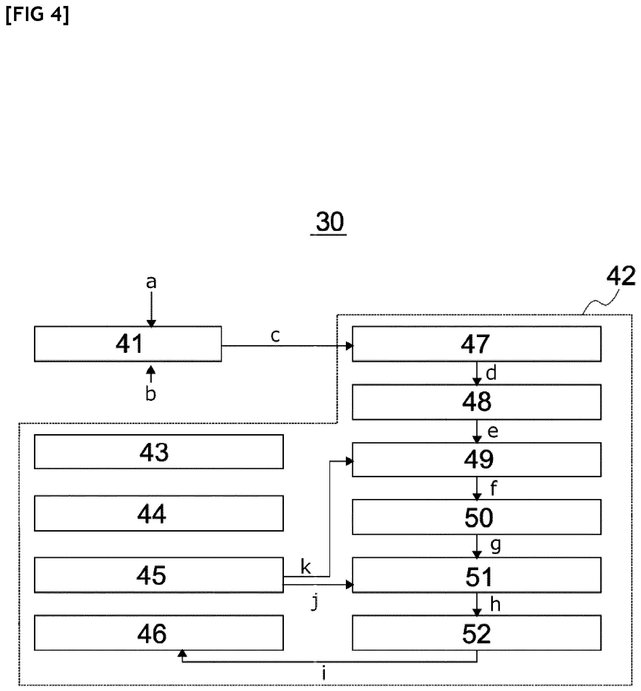

Fig. 4 is a functional block diagram of the composition inference device 30 according to the embodiment of the present invention. The composition inference device 30 includes a mass spectrometer unit 41 that performs mass spectrometry on all samples, and an information processing unit 42 that processes mass spectra acquired by the mass spectrometer unit 41.

The mass spectrometer unit 41 has a configuration including the mass spectrometer 31 and corresponds to a function realized by causing a controller 43 described later to control the mass spectrometer 31.

The mass spectrometer unit 41 ionizes gas components generated as a result of thermal desorption and/or pyrolysis on a loaded sample set (samples, learning samples, and background samples indicated by sign a in Fig. 4) while heating the sample set, performs mass spectrometry sequentially, and outputs mass spectra (sign c in Fig. 4).

At this time, an environmental factor resulting from an environment where the mass spectrometer 31 is installed (sign b in Fig. 4, which may be poly(dimethylsiloxane) generated from the pressure-reducing pump 32, for example) is further loaded simultaneously on the mass spectrometer 31 to exert influence on the mass spectra.

Described next is a function of each part of the information processing unit 42 having a configuration including the information processing device 33.

The controller 43 has a configuration including the processor 34. The controller 43 controls each part to realize each function of the composition inference device 30.

A storage part 44 has a configuration including the storage device 35. The storage part 44 includes a program stored in advance for control over each part, and provides a transitory or non-transitory storage area for data input to and output from each part. The storage part 44 is controlled by the controller 43 and is capable of reading and writing data.

An input part 45 has a configuration including the input device 37. An output part 46 has a configuration including the display device 36. The controller 43 controls these parts, thereby allowing input from a user of the composition inference device 30 to be accepted and the accepted input to be stored into the storage part 44, allowing information in the storage part 44 to be transmitted to each part, and allowing the transmitted information to be presented to the user of the composition inference device 30 through the output part 46.

A data matrix generating part 47 is a function realized by causing the controller 43 to execute the program stored in the storage part 44.

The data matrix generating part 47 generates a data matrix X from a two-dimensional mass spectrum including the mass spectrum (sign c in Fig. 4) in each row acquired for each heating temperature by the mass spectrometer unit 41, and transfers the data matrix X to an NMF processing part 48 described later (sign d in Fig. 4). The details of the data matrix X are as have already been described.

The NMF processing part 48 is a function realized by causing the controller 43 to execute the program stored in the storage part 44.

The NMF processing part 48 performs non-negative matrix factorization (NMF) on the data matrix X (sign d in Fig. 4) generated by the data matrix generating part 47 to factorize the data matrix X into the product of a normalized base spectrum matrix (S) and a corresponding intensity distribution matrix (C) ($X = C \times S^T$). Result thereof is transferred to a correction processing part 49 (sign e in Fig. 4). The details of the NMF process are as have already been described.

The correction processing part 49 is a function realized by causing the controller 43 to execute the program stored in the storage part 44.

The correction processing part 49 is the function of correcting the intensity distribution matrix through analysis on canonical correlation between the base spectrum matrix generated by the NMF processing part 48 and the data matrix, thereby generating a corrected intensity distribution matrix. The generated corrected intensity distribution matrix is transferred to a vector processing part 50 (sign f in Fig. 4).

The correction processing part 49 may further make the intensity correction already described in addition to the foregoing correction through the canonical correlation analysis. In this case, if there is a composition of a calibration sample (calib sample composition) to be used for the intensity correction, this composition is transferred to the correction processing part 49 through the input part 45 (sign k in Fig. 4). The details of the correction through the canonical correlation analysis (CCA filter) and the intensity correction are as have already been described.

The vector processing part 50 is a function realized by causing the controller 43 to execute the program stored in the storage part 44.

The vector processing part 50 partitions the corrected intensity distribution matrix generated by the correction processing part 49 into a submatrix corresponding to each of all the samples, and expresses all the samples in vector space using the submatrix as a feature vector. Result thereof is transferred to an end member determining part 51 (sign g in Fig. 4).

The end member determining part 51 is a function realized by causing the controller 43 to execute the program stored in the storage part 44.

The end member determining part 51 defines a K-1 dimensional simplex including all the feature vectors generated by the vector processing part 50, and determines K end members in the K-1 dimensional simplex. Result thereof is transferred to a content ratio calculating part 52 (sign h in Fig. 4).

At this time, if the learning sample contains an end member and has a determination label, information about the determination label is transferred from the input part 45 to the end member determining part 51 (sign j in Fig. 4).

As has already been described, however, the determination label is not essential for determining an end member (for processing by the end member determining part 51).

The content ratio calculating part 52 is a function realized by causing the controller 43 to execute the program stored in the storage part 44.

The content ratio calculating part 52 calculates a Euclidean distance between each of the K end members determined by the end member determining part 51 and the feature vector of an inference target sample generated by the vector processing part 50, and infers content ratios of the K components in the inference target sample on the basis of a ratio of the Euclidean distance between each of the K end members and the feature vector of the sample. Result of the content ratio inference is output from the output part 46 (sign i in Fig. 4).

At this time, the composition inference result about the learning sample may be output together.

Even if a sample is a solid sample hard to dissolve in a solvent and hard to undergo conventional composition analysis, the present composition inference device still makes it possible to make this sample available for analysis without particular preparatory process and to acquire accurate quantitative analysis result. The present composition inference device achieves significant reduction in time required for obtaining result, particularly by being applied to quality control of a sample as a mixture of a plurality of components, investigation of a cause for failure, etc.

Examples

The present composition analysis method will be described in detail on the basis of examples. The composition analysis method of the present invention should not be interpreted in a restricted sense by the following specific examples.

[Example 1]

(Preparation of Sample)

Three types of polymer dioxane solutions (about 4% by mass containing about 0.4 mg of a polymer solid content) including poly(methyl methacrylate) (symbol "M"), polystyrene (symbol "S"), and poly(ethyl methacrylate) (symbol "E") were casted on a sample pot and air-dried overnight, thereby obtaining samples. Table 4 shows the respective compositions of the samples. Mass spectrometry was performed using LCMS-2020 available from SHIMADZU CORPORATION as a detector, a DART ion source available from IonSense Inc., and ionRocket available from BioChromato, Inc. as a heater for the samples.

[Table 4]

| SampleName | Composition | | |
|---|---|---|---|
| | M | S | E |
| M | 1.000 | | |
| S | | 1.000 | |
| E | | | 1.000 |

(continued)

| | Composition | | |
|---|---|---|---|
| SampleName | M | S | E |
| EM | 0.475 | | 0.525 |
| MS | 0.510 | 0.490 | |
| SE | | 0.502 | 0.498 |
| MS01 | 0.884 | 0.116 | |
| MS005 | 0.958 | 0.042 | |
| MS001 | 0.990 | 0.010 | |
| MS0001 | 0.991 | 0.009 | |
| M1_S2_E2 | 0.201 | 0.397 | 0.402 |
| M2_S1_E2 | 0.360 | 0.228 | 0.413 |
| M2_S2_E1 | 0.408 | 0.394 | 0.197 |
| MSE_calib | 0.337 | 0.326 | 0.337 |

The resultant mass spectra were subjected to analysis using an algorithm that implements the following (i) to (iv):

(i) Soft orthogonal constraint on matrix C ARD and matrix S;
(ii) Merging condition in NMF optimization;
(iii) Removal of NMF artifact, background, and contamination using CCA-filter; and
(iv) Quantitative analysis without requiring calibration by incorporating environmental variable into correction term.

Hyper parameters used in the analysis are as follows:

NMF:
Orthogonal constrain weight w = 0.2, initial component number K = 50, small positive number a = $1 + 0.1^{-15}$, iteration number L = 2000, threshold = 0.9
CCA-Filter:

$$t_1 = 0.8,\ t_2 = 0.2.$$

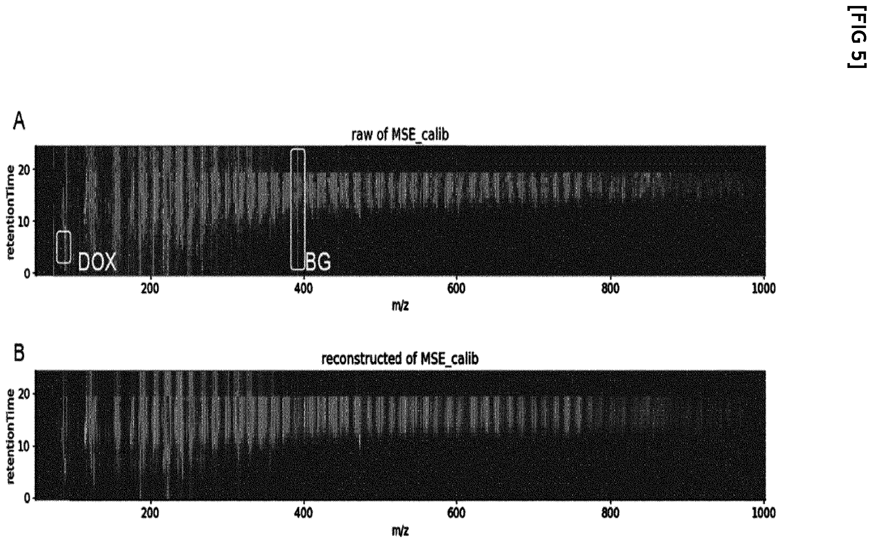

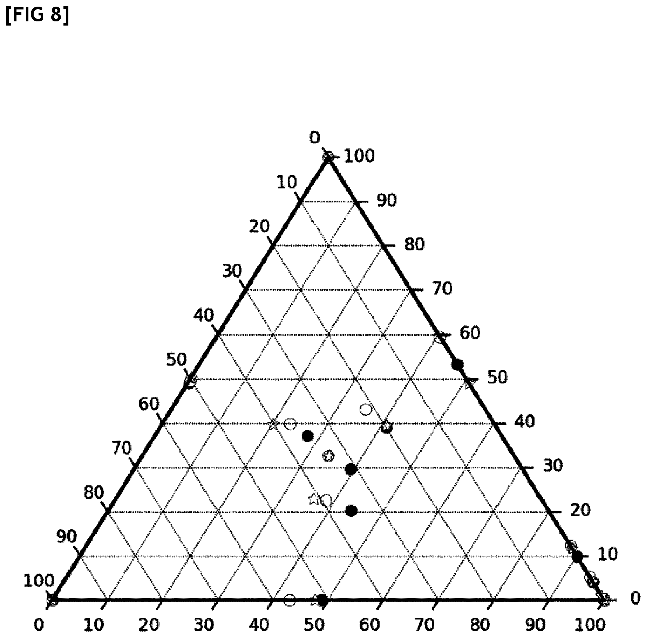

Fig. 5 is a view showing an original spectrum (A) of "MSE_calib" and a spectrum (B) reconstructed after performing the foregoing process. These spectra are found to have waveforms similar to each other. Specifically, it was found that, as a result of the foregoing process, a feature in the original spectrum is retained to provide data easily available for composition analysis.
While the original data contains background observed at 390 m/z that may be phthalate ester exhausted from an air conditioner, for example (a portion indicated as "BG" in Fig. 5(A)), this is removed from the reconstructed peak (B). Dioxane (a portion indicated as "DOX" in Fig. 5(A)) as a solvent used in the sample preparation was also removed. They each correspond to noise and such noise was found to be removed effectively by the present method.

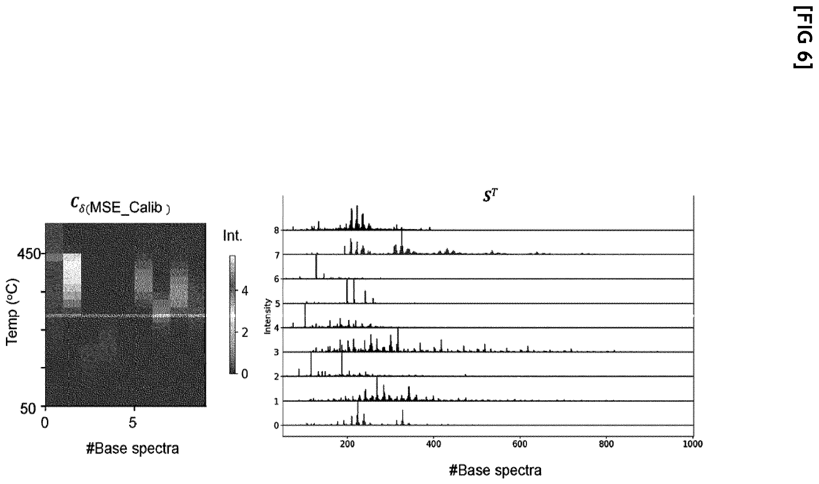

Fig. 6 shows $C_{\delta(MSE\_Calib)}$ as part of the matrix C after the NMF factorization and $S^T$ resulting from removing a background spectrum and impurity spectra using the CCA-Filter.

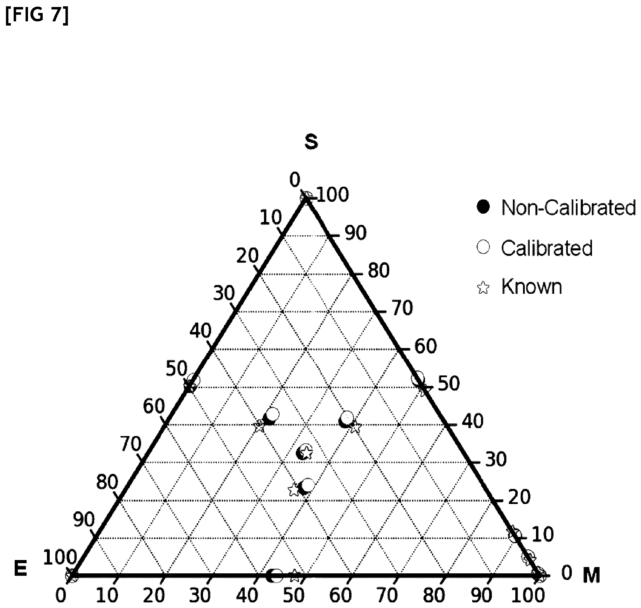

Fig. 7 shows composition analysis result about the ternary system composed of M, S, and E. Hollow circles and an explanatory note "Calibrated" in Fig. 7 represent composition inference result about a sample obtained using MSE_calib as a calibration sample, specifically, by providing composition information of MSE_calib as known information and making intensity correction on an intensity distribution matrix.
In Fig. 7, solid circles and an explanatory note "Non-calibrated" represent composition inference result about a sample obtained by making intensity correction on an intensity distribution matrix using the product of an environmental variable (specifically 610 m/z: peak intensity in poly(dimethylsiloxane)) and a sample mass.
In Fig. 7, "Known" in the legend, indicated by a star symbol, represents an actual composition (true value).
It is found from the result shown in Fig. 7 that excellent inference result similar to a true value was obtained in each of the cases "Calibrated" and "Non-calibrated."
Referring to Fig. 8 next, it shows composition inference result obtained by eliminating the orthogonal constraint in (1) from an NMF condition. Each plot is defined in the same way as in Fig. 7.

It was found from the result in Fig. 8 that the presence of the orthogonal constraint on the NMF provides more excellent inference result.

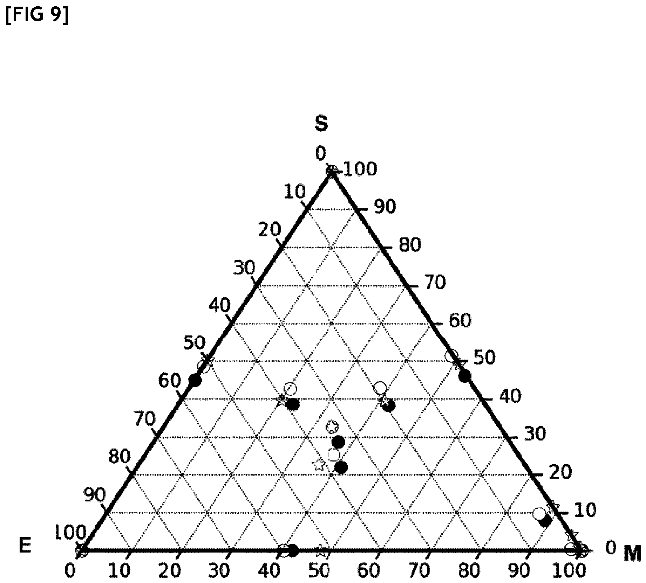

Referring to Fig. 9 next, it shows composition inference result obtained by eliminating only merging S from a condition for NMF optimization. Each plot is defined in the same way as in Fig. 7.

It was found from the result in Fig. 9 that the presence of merging S in a condition for the NMF optimization provides more excellent inference result.

While the number of components can be reduced only to 36 by the ARD, maintaining the Merging S condition allows an imposition to be inferred more precisely with components of a number 9. In this way, the significance of Merging S was proved.

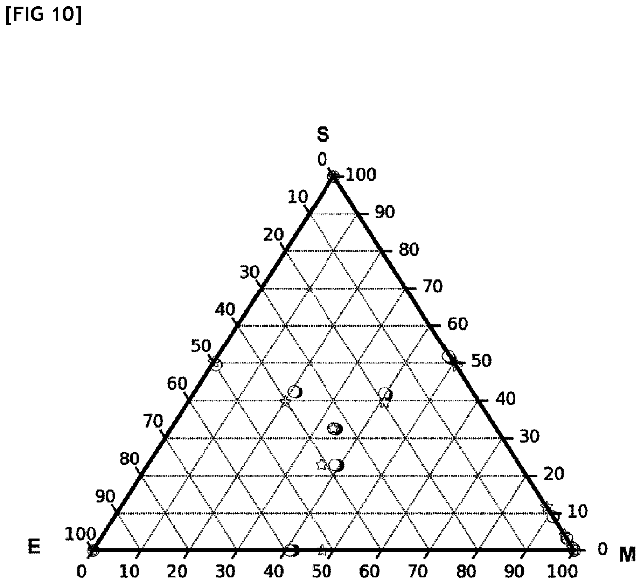

Fig. 10 shows composition inference result corresponding to result obtained by eliminating the CCA-filter (Comparative Example). Each plot is defined in the same way as in Fig. 7.

In Fig. 10, calculation result about a sample "SE" shows that about 1% of M was contained even though the content of M is 0%. Estimating 0% to be 1% is not practical as it lacks even a small percent of inference precision.

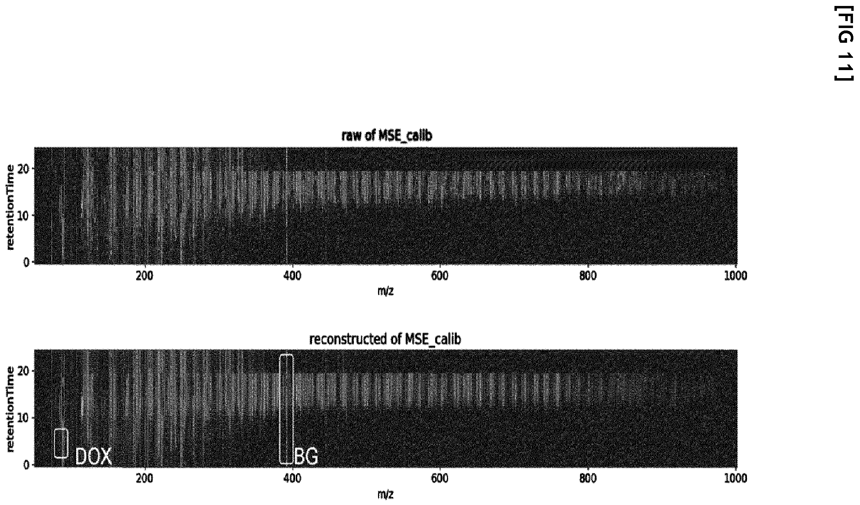

Fig. 11 is a view showing an original spectrum (raw of MSE_calib in the upper section) of "MSE_calib" and a spectrum (reconstructed of MSE_calib in the lower section) reconstructed after eliminating the CCA-filter and then performing the foregoing process.

It was found from this result that eliminating the CCA-filter leads to the failure of removing both the DOX noise and the BG noise (see the explanation about Fig. 5).

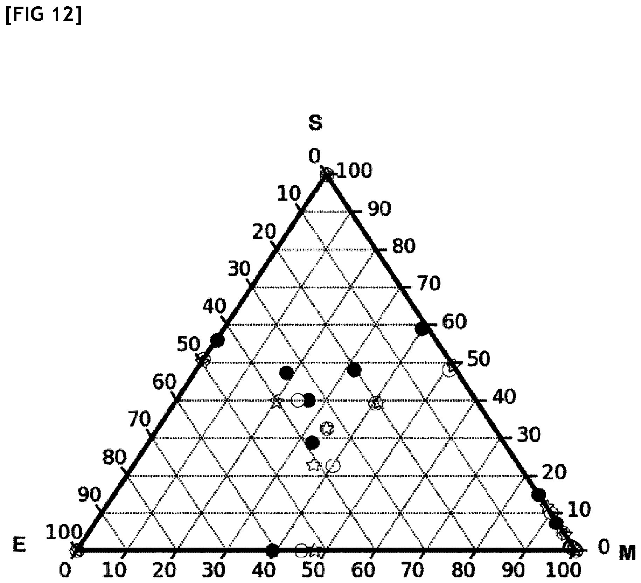

Fig. 12 shows composition inference result obtained without making correction using an environmental variable. In the result of Fig. 12, "Non-calibrated" indicated by solid circles largely deviates from Known, showing that making correction using an environmental variable provides more accurate inference result.

[Example 2]

(Inference Using Sample Not Containing End Member)

To determine that inference can be made precisely even if samples (sample group) including a learning sample do not contain an end member, samples listed in Table 5 were prepared and a test was conducted in the same way as in the Example 1.

In the table, symbols "M," "S," and "E" have the same meanings as those in Table 4 (Example 1). Each sample is prepared in such a manner that the weight fraction of a single component (polymer) does not exceed 80%.

[Table 5]

| sample | M(mass%) | S(mass%) | E(mass%) | mass(mg) |
|--------|----------|----------|----------|----------|
| 1 | 0 | 50 | 50 | 0.42 |
| 2 | 0 | 62 | 38 | 0.60 |
| 3 | 0 | 31 | 69 | 0.44 |
| 4 | 0 | 79 | 21 | 0.35 |
| 5 | 0 | 32 | 68 | 0.96 |
| 6 | 0 | 20 | 80 | 0.87 |
| 7 | 14 | 72 | 14 | 0.44 |
| 8 | 15 | 15 | 70 | 0.68 |
| 9 | 19 | 0 | 81 | 0.39 |
| 10 | 20 | 40 | 40 | 0.44 |
| 11 | 25 | 75 | 0 | 0.35 |
| 12 | 29 | 0 | 71 | 0.42 |
| 13 | 29 | 71 | 0 | 0.72 |
| 14 | 34 | 33 | 34 | 0.39 |
| 16 | 36 | 23 | 41 | 0.47 |
| 17 | 41 | 39 | 20 | 0.44 |

(continued)

| sample | M(mass%) | S(mass%) | E(mass%) | mass(mg) |
|---|---|---|---|---|
| 18 | 48 | 0 | 52 | 0.43 |
| 19 | 49 | 51 | 0 | 0.32 |
| 20 | 64 | 36 | 0 | 0.43 |
| 21 | 67 | 0 | 33 | 0.36 |
| 22 | 70 | 30 | 0 | 0.98 |
| 23 | 79 | 9 | 12 | 0.46 |
| 24 | 80 | 0 | 20 | 0.27 |
| 25 | 83 | 17 | 0 | 0.34 |

A two-dimensional mass spectrum of each of these samples was acquired in the same way as in the Example 1 and converted to a data matrix. The resultant data matrix was subjected to the first NMF process already described, namely,

[Formula 225]

$$X \approx AS$$

.

For estimation of a variance-covariance matrix of noise, calculation can be made only using X. Thus, the estimation was made as process inside a module in the first NMF process.

Next, A was subjected to intensity correction using the CCA-Filter, a weight, or a reference peak. As temperature distribution information is unnecessary for composition analysis, a total sum along a temperature axis may be obtained as follows for each sample:

[Formula 226]

$$A \in \mathbb{R}_+^{NT \times M} \longrightarrow \widetilde{A} \in \mathbb{R}_+^{N \times M}$$

.

Next, the following second NMF process was performed:

[Formula 227]

$$\widetilde{A} \approx CB$$

.

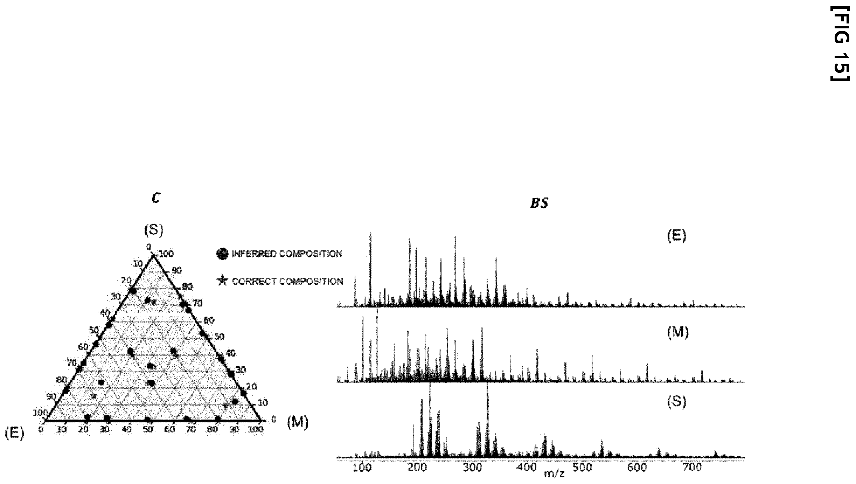

Evaluation and others using Riemannian metrics were performed as internal processing in the second NMF process. Fig. 15 shows C and BS illustrated as result of this processing.

Regarding C in the drawing, compositions inferred by the present method are indicated by circles and compositions of used samples (correct compositions) are indicated by stars. All these compositions exhibit favorable match therebetween.

Here, the absence of a plot at each vertex of C shows that the samples used herein do not contain an end member, namely, do not contain pure polymers of E, S, and M.

Regarding BS in the drawing, spectra of pure polymers of E, M, and S acquired through inference and actually-measured spectra of E, M, and S are superimposed on each other. There is no difference therebetween, proving that the interference including that of absolute intensity was made favorably.

As described above, as shown in BS in Fig. 15, the method of the present invention was confirmed to achieve accurate inference of spectra of pure polymers of E, M, and S and a composition of a mixture.

In the presence of test data, $X_{test}$ is projected onto S and B continuously to obtain Ctest.

[Example 3]

(Inference Using Sample Containing One End Member)

Next, it was confirmed that, if one of (learning) samples contains an end member, a component content in the other sample is determined arbitrarily (the end member is not required to be in a region external to a circle inscribed in a K-1 dimensional simplex).

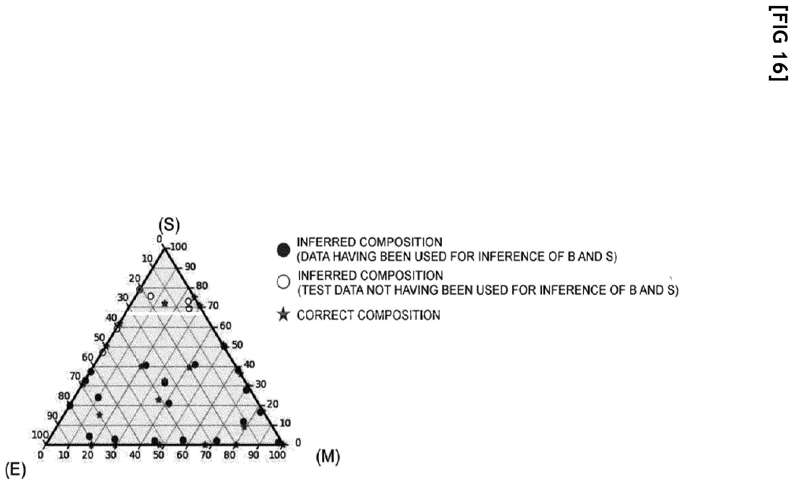

Composition analysis was conducted in the same way as in the Example 2 except that samples containing S having a weight percent equal to or greater than 40 were excluded from the samples listed in Table 5 and that M pure polymer was added as a sample. Result thereof is shown in Fig. 16.

In Fig. 16, dots indicated by solid circles represent inference result about samples used for inference of B and S, and stars indicate actual compositions of these samples. As seen from this result, it was confirmed that, while no sample of S is present in a region external to an inscribed circle (hollow circles represent samples not used for the inference), inference can be made precisely.

One of possible reasons therefor is that the following basis non-orthogonal term was incorporated as a penalty term into the second NMF process (Eq. 11) already described:

[Formula 228]

$$nonorth(\hat{B})$$

.

Industrial Applicability

In many cases of material development, material performance is desired to be examined by being reduced into each constituent component. While this requires identification and quantification of a component, increasing complexity of a system increases the difficulty levels thereof. High-resolution mass spectrometry in which peaks of different components are unlikely to overlap each other can be said to be the most powerful analysis method for identifying the chemical constitution of a component in a complicated mixture system. On the other hand, the mass spectrometry has a problem that it lacks in quantitativeness. Furthermore, while the mass spectrometry does not require separation by nature, a resultant spectrum is complicated and is likely to be unreadable in reality.

According to the technique developed by the present invention, a fragment of a material is directly subjected to pyrolysis in an atmosphere directly without preparatory process, resultant gas is analyzed through the DART-MS, and a resultant spectrum is learned without a teacher, thereby inferring pure end member spectra of respective components and representing a spectrum of a sample using a linear sum of the spectra. Even in the case of a material having a complicated composition, this technique still allows identification and quantification of a component in the material without requiring separation.

Regarding identification, as long as it is possible to acquire a highly-readable end member spectrum from which peaks derived from different components have been excluded completely, a subsequent procedure can be followed relatively easily. On the other hand, the present invention also encounters the fact that ionization efficiency differs between peaks and a peak intensity and an abundance ratio thereof do not proportionate to each other, which becomes a hindrance to quantitative analysis. In this regard, according to the present invention, it is possible to infer each peak intensity in an end member spectrum not as a normalized relative value but as an absolute intensity. In expressing a sample spectrum using a linear sum of end member spectra, this allows a linear coefficient of the spectrum to be determined as it is as a composition ratio.

The present invention, according to which a sample is decomposed into each component and a content ratio of the component is determined, can clearly be a useful tool in material development in terms of identification of a key component as a factor for exhibiting the performance of a material. The present invention is further expected to be applied in a considerably wide range of fields including quality control such as inspection on the content of a degraded component, compliance inspection on the content of a dangerous substance, etc., and inspection for detecting infringement. The present invention is further expected to accelerate techniques such as prediction of degeneration or lifetime of resin, detection of an infinitesimal impurity component in resin or organic materials, quantitative analysis on smell or taste, and traceability and tags for preventing false origin designation of food.

Furthermore, Plastics Europe submitted a suggestion to European Commission in September, 2021 to make it mandatory to achieve a 30% content of recycled plastic in plastic containers and packages until 2030. While use of recycled materials has been facilitated as understood therefrom, a general technique of measuring a content has not been established. The present invention is further expected to function as a technique of allowing accurate measurement of a ratio between a virgin material and a recycled material and to be applied in quality control, compliance inspection, and others.

Reference Signs List

10: K-1 dimensional simplex
12: Inscribed hypersphere
13-15: End member
16-18: Learning sample
19: Region
30: Composition inference device
31: Mass spectrometer
32: Pressure-reducing pump
33: Information processing device
34: Processor
35: Storage device
36: Display device
37: Input device
41: Mass spectrometer unit
42: Information processing unit
43: Controller
44: Storage part
45: Input part
46: Output part
47: Data matrix generating part
48: NMF processing part
49: Correction processing part
50: Vector processing part
51: End member determining part
52: Content ratio calculating part

**Claims**

1. A method of inferring a content ratio of a component in an inference target sample containing at least one type of the component selected from K types of components while K is an integer equal to or greater than 1, the method comprising:

   preparing learning samples of a number equal to or greater than K containing at least one type of component selected from the K types of components and having compositions different from each other, and a background sample not containing the component;

sequentially ionizing gas components generated by thermal desorption and/or pyrolysis while heating each sample in a sample set including the inference target sample, the learning samples, and the background sample, and observing mass spectra continuously;

storing, into respective rows, mass spectra acquired for a plurality of heating temperatures to acquire two-dimensional mass spectra of the respective samples, and merging at least two or more of the two-dimensional spectra and converting the spectra into a data matrix;

performing NMF process by which the data matrix is subjected to non-negative matrix factorization to be factorized into the product of a normalized base spectrum matrix and a corresponding intensity distribution matrix;

extracting a noise component in the intensity distribution matrix through analysis on canonical correlation between the base spectrum matrix and the data matrix, and correcting the intensity distribution matrix so as to reduce influence by the noise component, thereby acquiring a corrected intensity distribution matrix;

partitioning the corrected intensity distribution matrix into a submatrix corresponding to each of the samples, and expressing each of the samples in vector space using the submatrix as a feature vector;

defining a K-1 dimensional simplex including all of the feature vectors and determining K end members in the K-1 dimensional simplex; and

calculating a Euclidean distance between each of the K end members and the feature vector of the inference target sample, and inferring a content ratio of the component in the inference target sample on the basis of a ratio of the Euclidean distance, wherein

if the K is equal to or greater than 3, at least one of the feature vectors of the learning samples is present in each region external to a hypersphere inscribed in the K-1 dimensional simplex or the learning samples contain at least one of the end members.

2. The method according to claim 1, wherein

the K is an integer equal to or greater than 2, and
the inference target sample is a mixture of the components.

3. The method according to claim 1 or 2, wherein
the learning sample contains the end member.

4. The method according to claim 3, wherein
the end member is determined on the basis of a determination label given to the learning sample.

5. The method according to claim 3, wherein
the end member is determined through vertex component analysis on the feature vector of the learning sample.

6. The method according to claim 1, wherein
the end member is determined on the basis of an algorithm by which a vertex is defined in such a manner that the K-1 dimensional simplex has a minimum volume.

7. The method according to claim 6, wherein
the end member is determined by second NMF process by which the corrected intensity distribution matrix is subjected to non-negative matrix factorization to be factorized into the product of a matrix representing the weight fractions of the K types of components in the sample and a matrix representing an individual fragment abundance of each of the K types of components.

8. The method according to claim 6 or 7, wherein
the learning sample does not contain the end member.

9. The method according to any one of claims 1 to 8, wherein
acquiring the corrected intensity distribution matrix further includes making intensity correction on the intensity distribution matrix.

10. The method according to claim 9, wherein

the sample set further includes a calibration sample,
the calibration sample contains all the K types of components and has a known composition, and
the intensity correction includes normalizing the intensity distribution matrix.

**11.** The method according to claim 9, wherein

the intensity correction includes allocating at least part of the intensity distribution matrix using the product of the mass of the corresponding sample in the sample set and an environmental variable, and
the environmental variable is a variable representing influence on ionization efficiency of the component during the observation.

**12.** The method according to claim 11, wherein
the environmental variable is a total value of peaks in a mass spectrum of a compound having a molecular weight of 50-1500 contained in a predetermined quantity in an atmosphere during the observation or an organic low-molecular compound having a molecular weight of 50-500 contained in a predetermined quantity in each sample in the sample set.

**13.** A composition inference device that infers a content ratio of a component in an inference target sample containing at least one type of the component selected from K types of components while K is an integer equal to or greater than 1, the composition inference device comprising:

a mass spectrometer that sequentially ionizes gas components generated by thermal desorption and/or pyrolysis while heating each of samples in a sample set including learning samples of a number equal to or greater than K containing at least one type of component selected from the K types of components and having compositions different from each other, a background sample not containing the component, and the inference target sample, and observes mass spectra continuously; and
an information processing device that processes the observed mass spectra, wherein
the information processing device comprises:

a data matrix generating part that stores, into respective rows, mass spectra acquired for a plurality of heating temperatures to acquire two-dimensional mass spectra of the respective samples, and merges at least two or more of the two-dimensional spectra and converts the spectra into a data matrix;
an NMF processing part that performs NMF process by which the data matrix is subjected to non-negative matrix factorization to be factorized into the product of a normalized base spectrum matrix and a corresponding intensity distribution matrix;
a correction processing part that extracts a noise component in the intensity distribution matrix through analysis on canonical correlation between the base spectrum matrix and the data matrix, and corrects the intensity distribution matrix so as to reduce influence by the noise component, thereby generating a corrected intensity distribution matrix;
a vector processing part that partitions the corrected intensity distribution matrix into a submatrix corresponding to each of the samples in the sample set, and expresses each of the samples in vector space using the submatrix as a feature vector;
an end member determining part that defines a K-1 dimensional simplex including all of the feature vectors and determines K end members in the K-1 dimensional simplex; and
a content ratio calculating part that calculates a Euclidean distance between each of the K end members and the feature vector of the inference target sample, and infers a content ratio of the component in the inference target sample on the basis of a ratio of the Euclidean distance, and
if the K is equal to or greater than 3, at least one of the feature vectors of the learning samples is present in each region external to a hypersphere inscribed in the K-1 dimensional simplex or the learning samples contain at least one of the end members.

**14.** The composition inference device according to claim 13, wherein
the end member determining part determines the end member on the basis of a determination label given to the learning sample.

**15.** A program used in a composition inference device that infers a content ratio of a component in an inference target sample containing at least one type of the component selected from K types of components while K is an integer equal to or greater than 1, the composition inference device comprising:

a mass spectrometer that sequentially ionizes gas components generated by thermal desorption and/or pyrolysis while heating each of samples in a sample set including learning samples of a number equal to or greater than K containing at least one type of component selected from the K types of components and having compositions different from each other, a background sample not containing the component, and the inference target sample, and observes mass spectra continuously; and

an information processing device that processes the observed mass spectra,

the program comprising:

a data matrix generating function of storing, into respective rows, mass spectra acquired for a plurality of heating temperatures by the mass spectrometer to acquire two-dimensional mass spectra of the respective samples, and merging at least two or more of the two-dimensional spectra and converting the spectra into a data matrix;

an NMF processing function of performing NMF process by which the data matrix is subjected to non-negative matrix factorization to be factorized into the product of a normalized base spectrum matrix and a corresponding intensity distribution matrix;

a correction processing function of extracting a noise component in the intensity distribution matrix through analysis on canonical correlation between the base spectrum matrix and the data matrix, and correcting the intensity distribution matrix so as to reduce influence by the noise component, thereby generating a corrected intensity distribution matrix;

a vector processing function of partitioning the corrected intensity distribution matrix into a submatrix corresponding to each of the samples, and expressing each of the samples in vector space using the submatrix as a feature vector;

an end member determining function of defining a K-1 dimensional simplex including all of the feature vectors and determining K end members in the K-1 dimensional simplex; and

a content ratio calculating function of calculating a Euclidean distance between each of the K end members and the feature vector of the inference target sample, and inferring a content ratio of the component in the inference target sample on the basis of a ratio of the Euclidean distance, wherein

if the K is equal to or greater than 3, at least one of the feature vectors of the learning samples is present in each region external to a hypersphere inscribed in the K-1 dimensional simplex or the learning samples contain at least one of the end members.

## Patentansprüche

1. Verfahren zum Ableiten eines Anteilsverhältnisses einer Komponente in einer Auswertungszielprobe, die mindestens eine Art der Komponente enthält, die aus K Arten von Komponenten ausgewählt ist, wobei K eine ganze Zahl gleich oder größer als 1 ist, wobei das Verfahren Folgendes umfasst:

Vorbereiten von Lernproben in einer Anzahl gleich oder größer als K, die mindestens eine aus den K Arten von Komponenten ausgewählte Komponentenart enthalten und unterschiedliche Zusammensetzung aufweisen, sowie einer Hintergrundprobe, die die Komponente nicht enthält;

sequenzielles Ionisieren von durch thermische Desorption und/oder Pyrolyse erzeugten Gaskomponenten, während jede Probe in einem Probensatz, der die Auswertungszielprobe, die Lernproben und die Hintergrundprobe umfasst, erhitzt wird, und kontinuierliches Beobachten von Massenspektren;

Speichern der für eine Vielzahl von Erhitzungstemperaturen erfassten Massenspektren in jeweiligen Zeilen, um zweidimensionale Massenspektren der jeweiligen Proben zu erhalten, sowie Zusammenführen von mindestens zwei oder mehreren der zweidimensionalen Spektren und Umwandeln der Spektren in eine Datenmatrix;

Durchführen eines NMF-Verfahrens, bei dem die Datenmatrix einer nicht-negativen Matrixfaktorisierung unterzogen wird, um in das Produkt einer normalisierten Basisspektrummatrix und einer entsprechenden Intensitätsverteilungsmatrix faktorisiert zu werden;

Extrahieren einer Rauschkomponente in der Intensitätsverteilungsmatrix durch Analyse der kanonischen Korrelation zwischen der Basisspektrummatrix und der Datenmatrix sowie Korrigieren der Intensitätsverteilungsmatrix, um den Einfluss der Rauschkomponente zu verringern, wodurch eine korrigierte Intensitätsverteilungsmatrix erhalten wird;

Aufteilen der korrigierten Intensitätsverteilungsmatrix in eine jeder der Proben entsprechende Untermatrix und Darstellen jeder der Proben im Vektorraum unter Verwendung der Untermatrix als Merkmalsvektor;

Definieren eines K-1-dimensionalen Simplex, der alle Merkmalsvektoren enthält, und Bestimmen von K Endelementen in dem K-1-dimensionalen Simplex; und

Berechnen eines euklidischen Abstands zwischen jedem der K Endelemente und dem Merkmalsvektor der Auswertungszielprobe sowie Ableiten eines Anteilsverhältnisses der Komponente in der Auswertungszielprobe auf der Grundlage eines Verhältnisses des euklidischen Abstands, wobei

wenn K gleich oder größer als 3 ist, mindestens einer der Merkmalsvektoren der Lernproben in jeder Region außerhalb einer in das K-1-dimensionale Simplex eingeschriebenen Hyperkugel vorhanden ist oder die Lernproben mindestens eines der Endelemente enthalten.

2. Verfahren nach Anspruch 1, wobei

K eine ganze Zahl ist, die gleich oder größer als 2 ist, und
die Auswertungszielprobe eine Mischung der Komponenten ist.

3. Verfahren nach Anspruch 1 oder 2, wobei
die Lernprobe das Endelement enthält.

4. Verfahren nach Anspruch 3, wobei
das Endelement auf der Grundlage einer der Lernprobe zugewiesenen Bestimmungsbezeichnung bestimmt wird.

5. Verfahren nach Anspruch 3, wobei
das Endelement durch eine Eckpunktkomponentenanalyse des Merkmalsvektors der Lernprobe bestimmt wird.

6. Verfahren nach Anspruch 1, wobei
das Endelement auf der Grundlage eines Algorithmus bestimmt wird, durch den ein Eckpunkt so definiert wird, dass das K-1-dimensionale Simplex ein minimales Volumen aufweist.

7. Verfahren nach Anspruch 6, wobei
das Endelement durch ein zweites NMF-Verfahren bestimmt wird, bei dem die korrigierte Intensitätsverteilungsmatrix einer nicht-negativen Matrixfaktorisierung unterzogen wird, um in das Produkt einer Matrix, die die Gewichtsanteile der K Arten von Komponenten in der Probe darstellt, und einer Matrix, die die individuelle Fragmenthäufigkeit jeder der K Arten von Komponenten darstellt, faktorisiert zu werden.

8. Verfahren nach Anspruch 6 oder 7, wobei
die Lernprobe das Endelement nicht enthält.

9. Verfahren nach einem der Ansprüche 1 bis 8, wobei
das Erhalten der korrigierten Intensitätsverteilungsmatrix ferner das Vornehmen einer Intensitätskorrektur an der Intensitätsverteilungsmatrix umfasst.

10. Verfahren nach Anspruch 9, wobei

der Probensatz ferner eine Kalibrierprobe umfasst,
die Kalibrierungsprobe alle K Arten von Komponenten enthält und eine bekannte Zusammensetzung aufweist, und
die Intensitätskorrektur das Normalisieren der Intensitätsverteilungsmatrix umfasst.

11. Verfahren nach Anspruch 9, wobei

die Intensitätskorrektur das Zuweisen mindestens eines Teils der Intensitätsverteilungsmatrix unter Verwendung des Produkts aus der Masse der entsprechenden Probe im Probensatz und einer Umgebungsvariablen umfasst, und
die Umgebungsvariable eine Variable ist, die den Einfluss auf die Ionisierungseffizienz der Komponente während der Beobachtung darstellt.

12. Verfahren nach Anspruch 11, wobei
die Umgebungsvariable ein Gesamtwert von Peaks in einem Massenspektrum einer Verbindung mit einem Molekulargewicht von 50-1500 ist, die während der Beobachtung in einer vorbestimmten Menge in der Atmosphäre enthalten ist, oder einer organischen niedermolekularen Verbindung mit einem Molekulargewicht von 50-500, die in jeder Probe des Probensatzes in einer vorbestimmten Menge enthalten ist.

13. Vorrichtung zur Zusammensetzungsauswertung, die ein Anteilsverhältnis einer Komponente in einer Auswertungszielprobe ableitet, die mindestens eine Art der Komponente enthält, die ausgewählt ist aus K Arten von Komponenten, wobei K eine ganze Zahl gleich oder größer als 1 ist, wobei die Vorrichtung zur Zusammensetzungsauswertung Folgendes umfasst:

ein Massenspektrometer, das Gaskomponenten, die durch thermische Desorption und/oder Pyrolyse erzeugt werden, sequenziell ionisiert, während jede der Proben in einem Probensatz erhitzt wird, der Lernproben in einer Anzahl gleich oder größer als K, die mindestens eine aus den K Arten von Komponenten ausgewählte Komponentenart enthalten und unterschiedliche Zusammensetzung aufweisen, eine Hintergrundprobe, die die Komponente nicht enthält, und die Auswertungszielprobe umfasst, und das Massenspektren kontinuierlich beobachtet; und

eine Informationsverarbeitungsvorrichtung, die die beobachteten Massenspektren verarbeitet, wobei

die Informationsverarbeitungsvorrichtung Folgendes umfasst:

einen Datenmatrix-Erzeugungsteil, der in jeweiligen Zeilen Massenspektren speichert, die für eine Vielzahl von Erhitzungstemperaturen erhalten werden, um zweidimensionale Massenspektren der jeweiligen Proben zu erhalten, und der mindestens zwei oder mehrere der zweidimensionalen Spektren zusammenführt und die Spektren in eine Datenmatrix umwandelt;

einen NMF-Verarbeitungsteil, der ein NMF-Verfahren durchführt, bei dem die Datenmatrix einer nicht-negativen Matrixfaktorisierung unterzogen wird, um in das Produkt einer normalisierten Basisspektrummatrix und einer entsprechenden Intensitätsverteilungsmatrix faktorisiert zu werden;

einen Korrekturverarbeitungsteil, der durch Analyse der kanonischen Korrelation zwischen der Basisspektrummatrix und der Datenmatrix eine Rauschkomponente in der Intensitätsverteilungsmatrix extrahiert und die Intensitätsverteilungsmatrix korrigiert, um den Einfluss der Rauschkomponente zu verringern, wodurch eine korrigierte Intensitätsverteilungsmatrix erzeugt wird;

einen Vektorverarbeitungsteil, der die korrigierte Intensitätsverteilungsmatrix in eine jeder der Proben im Probensatz entsprechende Untermatrix unterteilt, und jede der Proben im Vektorraum unter Verwendung der Untermatrix als Merkmalsvektor darstellt;

einen Endelement-Bestimmungsteil, der ein K-1-dimensionales Simplex definiert, das alle Merkmalsvektoren enthält, und K Endelemente in dem K-1-dimensionalen Simplex bestimmt; und

einen Anteilsverhältnis-Berechnungsteil, der einen euklidischen Abstand zwischen jedem der K Endelemente und dem Merkmalsvektor der Auswertungszielprobe berechnet und ein Anteilsverhältnis der Komponente in der Auswertungszielprobe auf der Grundlage eines Verhältnisses des euklidischen Abstands ableitet, und

wenn K gleich oder größer als 3 ist, mindestens einer der Merkmalsvektoren der Lernproben in jedem Bereich außerhalb einer in das K-1-dimensionale Simplex eingeschriebenen Hyperkugel vorhanden ist oder die Lernproben mindestens eines der Endelemente enthalten.

14. Vorrichtung zur Zusammensetzungsauswertung nach Anspruch 13, wobei der Endelement-Bestimmungsteil das Endelement auf der Grundlage einer der Lernprobe zugewiesenen Bestimmungsbezeichnung bestimmt.

15. Programm, das in einer Vorrichtung zur Zusammensetzungsauswertung verwendet wird, die ein Anteilsverhältnis einer Komponente in einer Auswertungszielprobe ableitet, die mindestens eine Art der Komponente enthält, die ausgewählt ist aus K Arten von Komponenten, wobei K eine ganze Zahl gleich oder größer als 1 ist, wobei die Vorrichtung zur Zusammensetzungsauswertung Folgendes umfasst:

ein Massenspektrometer, das sequenziell Gaskomponenten ionisiert, die durch thermische Desorption und/oder Pyrolyse erzeugt werden, während jede der Proben in einem Probensatz erhitzt wird, der Lernproben in einer Anzahl gleich oder größer als K, die mindestens eine aus den K Arten von Komponenten ausgewählte Komponentenart enthalten und unterschiedliche Zusammensetzungen aufweisen, eine Hintergrundprobe, die die Komponente nicht enthält, sowie die Auswertungszielprobe umfasst, und das die Massenspektren kontinuierlich beobachtet; und

eine Informationsverarbeitungsvorrichtung, die die beobachteten Massenspektren verarbeitet, wobei das Programm Folgendes umfasst:

eine Datenmatrix-Erzeugungsfunktion zum Speichern in jeweiligen Zeilen von Massenspektren, die für eine Vielzahl von Erhitzungstemperaturen durch das Massenspektrometer erfasst werden, um zweidimensionale Massenspektren der jeweiligen Proben zu erhalten, sowie zum Zusammenführen von mindestens zwei oder mehreren der zweidimensionalen Spektren und zum Umwandeln der Spektren in eine Datenmatrix; eine NMF-Verarbeitungsfunktion, die ein NMF-Verfahren durchführt, bei dem die Datenmatrix einer nichtnegativen Matrixfaktorisierung unterzogen wird, um in das Produkt einer normalisierten Basisspektrummatrix und einer entsprechenden Intensitätsverteilungsmatrix faktorisiert zu werden;

eine Korrekturverarbeitungsfunktion zum Extrahieren einer Rauschkomponente in der Intensitätsverteilungsmatrix durch Analyse der kanonischen Korrelation zwischen der Basisspektrummatrix und der Datenmatrix und zum Korrigieren der Intensitätsverteilungsmatrix, um den Einfluss der Rauschkomponente zu verringern, wodurch eine korrigierte Intensitätsverteilungsmatrix erzeugt wird;

eine Vektorverarbeitungsfunktion zum Aufteilen der korrigierten Intensitätsverteilungsmatrix in eine jeder der Proben entsprechende Untermatrix und zum Darstellen jeder der Proben im Vektorraum unter Verwendung der Untermatrix als Merkmalsvektor;

eine Endelement-Bestimmungsfunktion zum Definieren eines K-1-dimensionalen Simplex, der alle Merkmalsvektoren enthält, und zum Bestimmen von K Endelementen in dem K-1-dimensionalen Simplex; und eine Anteilsverhältnis-Berechnungsfunktion zum Berechnen eines euklidischen Abstands zwischen jedem der K Endelemente und dem Merkmalsvektor der Auswertungszielprobe sowie zum Ableiten eines Anteilsverhältnisses der Komponente in der Auswertungszielprobe auf der Grundlage eines Verhältnisses des euklidischen Abstands, wobei

wenn K gleich oder größer als 3 ist, mindestens einer der Merkmalsvektoren der Lernproben in jedem Bereich außerhalb einer in den K-1-dimensionalen Simplex eingeschriebenen Hyperkugel vorhanden ist oder die Lernproben mindestens eines der Endelemente enthalten.

**Revendications**

1. Procédé d'inférence d'un rapport de teneur en un composant dans un échantillon cible d'inférence contenant au moins un type dudit composant choisi parmi K types de composants, K étant un entier supérieur ou égal à 1, le procédé comprenant :

   la préparation d'échantillons d'apprentissage, en un nombre supérieur ou égal à K, contenant au moins un type de composant choisi parmi les K types de composants et ayant des compositions différentes les unes des autres, et d'un échantillon de base dépourvu du composant,

   l'ionisation séquentielle de composants gazeux générés par désorption thermique et/ou pyrolyse pendant un chauffage de chaque échantillon d'un ensemble d'échantillons comprenant l'échantillon cible d'inférence, les échantillons d'apprentissage et l'échantillon de base, et l'observation continue de spectres de masse,

   le stockage, dans des rangées respectives, des spectres de masse acquis pour une pluralité de températures de chauffe pour acquérir des spectres de masse bidimensionnels des échantillons respectifs et la fusion d'au moins deux des spectres bidimensionnels et la conversion des spectres en une matrice de données,

   la réalisation d'un processus NMF lors duquel la matrice de données est soumise à une factorisation matricielle non négative pour être factorisée en le produit d'une matrice de spectre de base normalisée et d'une matrice de distribution d'intensité correspondante,

   l'extraction d'une composante de bruit dans la matrice de distribution d'intensité par analyse de corrélation canonique entre la matrice de spectre de base et la matrice de données, et la correction de la matrice de distribution d'intensité pour réduire l'influence de la composante de bruit, pour ainsi acquérir une matrice de distribution d'intensité corrigée,

   le partitionnement de la matrice de distribution d'intensité corrigée en une sous-matrice correspondant à chacun des échantillons et l'expression de chacun des échantillons dans un espace vectoriel à l'aide de la sous-matrice en tant que vecteur caractéristique,

   la définition d'un simplexe de dimension K-1 comportant tous les vecteurs caractéristiques et la détermination de K composantes pures (endmembers) dans le simplexe de dimension K-1, et

   le calcul d'une distance euclidienne entre chacune des K composantes pures et le vecteur caractéristique de l'échantillon cible d'inférence, et l'inférence d'un rapport de teneur en composant dans l'échantillon cible d'inférence en fonction d'un rapport de la distance euclidienne, étant entendu que

   si K est supérieur ou égal à 3, au moins un des vecteurs caractéristiques des échantillons d'apprentissage est présent dans chaque région extérieure à une hypersphère inscrite dans le simplexe de dimension K-1 ou les échantillons d'apprentissage contiennent au moins une des composantes pures.

2. Procédé selon la revendication 1, dans lequel

K est un entier supérieur ou égal à 2, et

l'échantillon cible d'inférence est un mélange des composants.

**3.** Procédé selon la revendication 1 ou 2, dans lequel

l'échantillon d'apprentissage contient la composante pure.

**4.** Procédé selon la revendication 3, dans lequel

la composante pure est déterminée en fonction d'une étiquette de détermination donnée à l'échantillon d'apprentissage.

**5.** Procédé selon la revendication 3, dans lequel

la composante pure est déterminée par analyse des composantes extrêmes sur le vecteur caractéristique de l'échantillon d'apprentissage.

**6.** Procédé selon la revendication 1, dans lequel

la composante pure est déterminée en fonction d'un algorithme selon lequel une extrémité est définie de telle façon que le simplexe de dimension K-1 a un volume minimal.

**7.** Procédé selon la revendication 6, dans lequel

la composante pure est déterminée par un deuxième processus NMF lors duquel la matrice de distribution d'intensité corrigée est soumise à une factorisation matricielle non négative pour être factorisée en le produit d'une matrice représentant les fractions de poids des K types de composants de l'échantillon et d'une matrice représentant une abondance de fragments individuels de chacun des K types de composants.

**8.** Procédé selon la revendication 6 ou 7, dans lequel

l'échantillon d'apprentissage ne contient pas la composante pure.

**9.** Procédé selon l'une quelconque des revendications 1 à 8, dans lequel

l'acquisition de la matrice de distribution d'intensité corrigée comprend en outre la réalisation d'une correction d'intensité sur la matrice de distribution d'intensité.

**10.** Procédé selon la revendication 9, dans lequel

l'ensemble d'échantillons comprend en outre un échantillon d'étalonnage,

l'échantillon d'étalonnage contient tous les K types de composants et a une composition connue, et

la correction d'intensité comprend la normalisation de la matrice de distribution d'intensité.

**11.** Procédé selon la revendication 9, dans lequel

la correction d'intensité comprend l'attribution d'au moins une partie de la matrice de distribution d'intensité à l'aide du produit de la masse de l'échantillon correspondant au sein de l'ensemble d'échantillons et d'une variable environnementale, et

la variable environnementale est une variable représentant l'influence sur l'efficacité d'ionisation du composant pendant l'observation.

**12.** Procédé selon la revendication 11, dans lequel

la variable environnementale est une valeur totale des pics d'un spectre de masse d'un composé ayant un poids moléculaire de 50 à 1500 contenu dans une quantité prédéterminée dans une atmosphère pendant l'observation ou d'un composé organique de faible masse moléculaire ayant une masse moléculaire de 50 à 500 contenus dans une quantité prédéterminée dans chaque échantillon de l'ensemble d'échantillons.

**13.** Dispositif d'inférence de composition qui infère un rapport de teneur en un composant dans un échantillon cible d'inférence contenant au moins un type dudit composant choisi parmi K types de composants, K étant un entier supérieur ou égal à 1, le dispositif d'inférence de composition comprenant :

un spectromètre de masse qui ionise séquentiellement des composants gazeux générés par désorption thermique et/ou pyrolyse tout en chauffant chacun des échantillons d'un ensemble d'échantillons comprenant des échantillons d'apprentissage, en un nombre supérieur ou égal à K, contenant au moins un type de composant choisi parmi les K types de composants et ayant des compositions différentes les unes des autres, un échantillon de base dépourvu du composant et l'échantillon cible d'inférence, et qui observe les spectres de masse en continu, et

un dispositif de traitement d'informations qui traite les spectres de masse observés ;
ledit dispositif de traitement d'informations comprenant :

une partie génératrice de matrices de données qui stocke, dans des rangées respectives, les spectres de masse acquis pour une pluralité de températures de chauffe pour acquérir des spectres de masse bidimensionnels des échantillons respectifs, et fusionne au moins deux des spectres bidimensionnels et convertit les spectres en une matrice de données,

une partie traitement NMF qui réalise un processus NMF lors duquel la matrice de données est soumise à une factorisation matricielle non négative pour être factorisée en le produit d'une matrice de spectre de base normalisé et d'une matrice de distribution d'intensité correspondante,

une partie traitement de correction qui extrait une composante de bruit dans la matrice de distribution d'intensité par analyse de corrélation canonique entre la matrice de spectre de base et la matrice de données, et corrige la matrice de distribution d'intensité pour réduire l'influence de la composante de bruit, générant ainsi une matrice de distribution d'intensité corrigée,

une partie traitement de vecteur qui partitionne la matrice de distribution d'intensité corrigée en une sous-matrice correspondant à chacun des échantillons de l'ensemble d'échantillons et exprime chacun des échantillons dans un espace vectoriel à l'aide de la sous-matrice en tant que vecteur caractéristique,

une partie détermination de composante pure qui définit un simplexe de dimension K-1 comportant tous les vecteurs caractéristiques et détermine K composantes pures (endmembers) dans le simplexe de dimension K-1, et

une partie calcul de rapport de teneur qui calcule une distance euclidienne entre chacune des K composantes pures et le vecteur caractéristique de l'échantillon cible d'inférence, et infère un rapport de teneur en composant dans l'échantillon cible d'inférence en fonction d'un rapport de la distance euclidienne ; et

si K est supérieur ou égal à 3, au moins un des vecteurs caractéristiques des échantillons d'apprentissage est présent dans chaque région extérieure à une hypersphère inscrite dans le simplexe de dimension K-1 ou les échantillons d'apprentissage contiennent au moins une des composantes pures.

14. Dispositif d'inférence de composition selon la revendication 13, dans lequel
la partie détermination de composante pure détermine la composante pure en fonction d'une étiquette de détermination donnée à l'échantillon d'apprentissage.

15. Programme mis en œuvre dans un dispositif d'inférence de composition qui infère un rapport de teneur en un composant dans un échantillon cible d'inférence contenant au moins un type dudit composant choisi parmi les K types de composants, K étant un entier supérieur ou égal à 1, le dispositif d'inférence de composition comprenant :

un spectromètre de masse qui ionise séquentiellement des composants gazeux générés par désorption thermique et/ou pyrolyse tout en chauffant chacun des échantillons d'un ensemble d'échantillons comprenant des échantillons d'apprentissage, en un nombre supérieur ou égal à K, contenant au moins un type de composant choisi parmi les K types de composants et ayant des compositions différentes les unes des autres, un échantillon de base dépourvu du composant et l'échantillon cible d'inférence, et qui observe les spectres de masse en continu, et

un dispositif de traitement d'informations qui traite les spectres de masse observés ;
ledit programme comprenant :

une fonction génératrice de matrices de données consistant à stocker, dans des rangées respectives, les spectres de masse acquis pour une pluralité de températures de chauffe par le spectromètre de masse pour acquérir des spectres de masse bidimensionnels des échantillons respectifs, à fusionner au moins deux des spectres bidimensionnels et à convertir les spectres en une matrice de données,

une fonction traitement NMF consistant à réaliser un processus NMF lors duquel la matrice de données est soumise à une factorisation matricielle non négative pour être factorisée en le produit d'une matrice de spectre de base normalisé et d'une matrice de distribution d'intensité correspondante,

une fonction traitement de correction consistant à extraire une composante de bruit dans la matrice de distribution d'intensité par analyse de corrélation canonique entre la matrice de spectre de base et la matrice de données, et à corriger la matrice de distribution d'intensité pour réduire l'influence de la composante de bruit, générant ainsi une matrice de distribution d'intensité corrigée,

une fonction traitement de vecteur consistant à partitionner la matrice de distribution d'intensité corrigée en une sous-matrice correspondant à chacun des échantillons, et à exprimer chacun des échantillons dans un espace vectoriel à l'aide de la sous-matrice en tant que vecteur caractéristique,

une fonction détermination de composante pure consistant à définir un simplexe de dimension K-1 comportant tous les vecteurs caractéristiques et à déterminer K composantes pures (endmembers) dans le simplexe de dimension K-1, et

une fonction calcul de rapport de teneur consistant à calculer une distance euclidienne entre chacune des K composantes pures et le vecteur caractéristique de l'échantillon cible d'inférence, et à inférer un rapport de teneur en composant dans l'échantillon cible d'inférence en fonction d'un rapport de la distance euclidienne, étant entendu que

si K est supérieur ou égal à 3, au moins un des vecteurs caractéristiques des échantillons d'apprentissage est présent dans chaque région extérieure à une hypersphère inscrite dans le simplexe de dimension K-1 ou les échantillons d'apprentissage contiennent au moins une des composantes pures.

[FIG 1]

```
                    ┌──────────────────────┐
                    │        START         │
                    └──────────┬───────────┘
                               ▼
                    ┌──────────────────────┐
                    │        S101          │
                    └──────────┬───────────┘
                               ▼
                    ┌──────────────────────┐
                    │        S102          │
                    └──────────┬───────────┘
                               ▼
                    ┌──────────────────────┐
                    │        S103          │
                    └──────────┬───────────┘
                               ▼
                    ┌──────────────────────┐
                    │        S104          │
                    └──────────┬───────────┘
                               ▼
                    ┌──────────────────────┐
                    │        S105          │
                    └──────────┬───────────┘
                               ▼
                    ┌──────────────────────┐
                    │        S106          │
                    └──────────┬───────────┘
                               ▼
                    ┌──────────────────────┐
                    │        S107          │
                    └──────────┬───────────┘
                               ▼
                    ┌──────────────────────┐
                    │        S108          │
                    └──────────┬───────────┘
                               ▼
                    ┌──────────────────────┐
                    │         END          │
                    └──────────────────────┘
```

[FIG 2]

[FIG 3]

<u>30</u>

| 31 |
|----|
| 32 |

| 33 |
|----|
| 34 |
| 35 |
| 36 |
| 37 |

[FIG 4]

[FIG 5]

EP 4 361 624 B1

A       raw of MSE_calib

B       reconstructed of MSE_calib

[FIG 6]

[FIG 7]

[FIG 8]

[FIG 9]

[FIG 10]

[FIG 11]

[FIG 12]

[FIG 13]

[FIG 14]

[FIG 15]

101

[FIG 16]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070288174 A1
- US 20090121125 A1

**Non-patent literature cited in the description**

- *Analytical chemistry*, 2020, vol. 92 (2), 1925-1933
- **IVICA KOPRIVA** ; **IVANKA JERIC**. *Analytical Chemistry*, 2010, vol. 82, 1911-1920
- **SHIGA, M. et al.** Sparse modeling of EELS and EDX spectral imaging data by nonnegative matrix factorization. *Ultramicroscopy*, 2016, vol. 170, 43-59
- Robust Volume Minimization-based Matrix Factorization for remote Sensing and Document Clustering. *IEEE TRANSACTIONS ON SIGNAL PROCESSING*, 01 December 2016, vol. 64 (23)
- **S. BOYD** ; **N. PARIKH** ; **E. CHU** ; **B. PELEATO** ; **J. ECKSTEIN**. Distributed optimization and statistical learning via the alternating direction method of multipliers. *Found. Trends Mach. Learn*, 2010, vol. 3, 1-122
- ROBUST VOLUME MINIMIZATION-BASED MATRIX FACTORIZATION VIA ALTERNATING OPTIMIZATION Department of ECE. **X. FU** ; **W. MA** ; **K. HUANG** ; **N. D. SIDIROPOULOS**. Icassp 2016. MI. University of Minnesota, 2016, 2534-2538
- **X. FU** ; **K. HUANG** ; **B. YANG** ; **W. K. MA** ; **N. D. SIDIROPOULOS**. Robust Volume Minimization-Based Matrix Factorization for Remote Sensing and Document Clustering. *IEEE Trans. Signal Process.*, 2016, vol. 64, 6254-6268
- **J. M. B. DIAS** ; **J. M. P. NASCIMENTO**. Vertex component analysis: A geometric-based approach to unmix hyperspectral data.. *Signal Image Process. Remote Sens.*, 2006, vol. 43, 415-439